# EUROPEAN PATENT SPECIFICATION

(11) **EP 1 460 984 B1**
(45) Date of publication and mention of the grant of the patent: **29.09.2010**
(21) Application number: 02789098.7
(22) Date of filing: 25.11.2002
(51) Int. Cl.: A61F 13/15

(54) **ABSORBENT ARTICLE WITH IMPROVED FIT**
SAUGFÄHIGER ARTIKEL MIT VERBESSERTEM SITZ
ARTICLE ABSORBANT A AJUSTEMENT AMELIORE

(30) Priority: 06.12.2001 SE 0104098
(43) Date of publication of application: 29.09.2004
(73) Proprietor: SCA Hygiene Products AB, 405 03 Göteborg (SE)
(72) Inventor: WIDLUND, Urban, S-435 43 Pixbo (SE); DREVIK, Solgun, S-435 35 Mölnlycke (SE); ASP, Fredrik, S-439 35 Onsala (SE); BOISSIER, Elisabeth, S-434 94 Vallda (SE)
(74) Representative: Olsson, Stefan
(86) International application number: PCT/SE2002/002152
(87) International publication number: WO 2003/053301

(56) References cited:
- EP-A2- 0 852 938
- EP-A2- 0 956 844
- EP-A2- 0 965 318
- WO-A1-99/25282
- US-A- 4 886 513
- US-B1- 6 210 385
- US-B1- 6 350 257

## Description

### TECHNICAL FIELD

The present invention relates to an absorbent article, such as a sanitary towel, a panty liner, an incontinent pad, a nappy or the like, which article has a longitudinal direction and a transverse direction, a front portion, a rear portion, a crotch portion located between the rear portion and the front portion, an absorbent element and a liquidtight layer, and also a stiffening element which is intended to contribute to the three-dimensional shape of the article during its use.

### BACKGROUND ART

A great many different demands are made of absorbent articles, such as a sanitary towel, an incontinence pad, a nappy or the like, which are not easy to satisfy simultaneously. A fundamental requirement is that the article, for example a sanitary towel, should be capable of catching and absorbing bodily fluid discharged from the wearer. Conventional sanitary towels in sizes intended for heavy flows of menstrual fluid have been of thick and relatively wide design. Sanitary towels of this type are described in, for example, US 3 294 091. Thick and relatively wide sanitary towels of this type theoretically have great absorption capacity but in practice, when the sanitary towel is subjected to compression forces when squeezed together between the thighs of the wearer, much of the take-up capacity and absorption capacity is lost. The sanitary towel is squeezed together into an arbitrary rope-like shape which frequently does not offer a sufficiently large receiving surface for the menstrual fluid discharged, and leakage occurs in the case of heavy flows of menstrual fluid. The sanitary towel can also be pressed together between the thighs of the wearer in such a manner that the side edges of the sanitary towel and the liquidtight layer are folded in over the liquid-permeable surface and in this way reduce the size of the liquid-receiving surface available.

Sanitary towels are intended to be positioned inside a pair of briefs, the design of which may vary. In this connection, sanitary towels can be positioned incorrectly inside the briefs. There is therefore a risk of the sanitary towel being, by mistake, positioned too far forward or too far back or displaced slightly in the lateral direction and therefore of the absorption capacity and receiving surface of the entire sanitary towel not being optimally utilized.

Conventional sanitary towels are generally retained in the briefs of the wearer by means of pressure-sensitive adhesive or friction coatings. The sanitary towel is fitted by the towel being put in place in the briefs, after which the latter are pulled up into position. When fitting the article inside the briefs, however, it is difficult to achieve a positioning which is optimum in relation to the body of the wearer. Use is usually made of the crotch portion of the briefs in order to determine where the sanitary towel should be positioned. As sanitary towels are manufactured in a great many sizes and models, the position and design of the crotch portion provide a particularly uncertain indication of where in the briefs a sanitary towel is to be positioned, and the functioning of the sanitary towel during use is consequently not always as desired.

Another cause of leakage occurring past sanitary towels attached inside the briefs of the wearer is that the sanitary towel moves together with the briefs instead of following the body movements of the wearer. This means that even a sanitary towel which was from the outset positioned correctly in the briefs in relation to the body can be pulled out of this position by the briefs.

In order to attempt to reduce leakage arising as a result of the sanitary towel being pressed together between the legs of the wearer, it has become usual to provide the sanitary towels with special attachment flaps. It is known from, for example, SE 455 668, US 4 285 343, EP 0 130 848, EP 0 134 086 and US 4 608 047 to provide sanitary towels with flexible side flaps or wings projecting from the longitudinal side edges. These are intended to be folded around the edge portions of the briefs of the wearer when the sanitary towel is put on, and to be attached to the outside of the briefs. The side flaps themselves constitute protection against side edge leakage and soiling of the briefs. Moreover, deformation of the absorption body of the sanitary towel is counteracted by virtue of the fact that the sanitary towel is anchored at the leg edges of the briefs and is held extended between these during use.

Document EP-A- 0965318 discloses a shaped element to be placed in an absorbent article and which is made of a rigid material. Said shaped element is provided with one cut out in its rear portion in order to increase adaptation of the absorbent article to the body as well as comfort for the wearer.

However, a considerable disadvantage of providing absorbent articles with such attachment flaps is that many wearers find it embarrassing that the attachment flaps are visible on the outside of the briefs. This also means that absorbent articles with such attachment flaps cannot be used when, for example, the wearer is wearing a swimsuit.

Another disadvantage of the attachment flaps is that they are relatively difficult to handle and require many manual operations in order to be fitted correctly around the leg edges of the briefs. Furthermore, especially in the case of attachment flaps which extend quite a long way along the side edges of a sanitary towel, it can be virtually impossible to fold the attachment flaps around the curved leg edges of the briefs without chafing and unattractive creases in the attachment flaps occurring.

A further problem of sanitary towels with attachment flaps is that the functioning of the attachment flaps or wings depends on the design of the briefs. It goes without saying that a sanitary towel with attachment flaps interacts differently with briefs with a wide crotch compared with briefs with a very narrow crotch.

Attachment flaps or wings on sanitary towels protect the leg edges of the briefs from soiling but, as emerged above, are far from being an entirely satisfactory solution.

In order to improve leakproofness, EP 0 067 465 has proposed manufacturing a two-part sanitary towel in which the two parts are interconnected only at their end portions. The lower part is fastened in the briefs of the wearer, and the upper part makes contact with the body of the wearer. The idea is that the parts will be able to move slightly in relation to one another during use. The mobility between the parts is, however, very limited, and the known sanitary towel is still dependent on the movements of the briefs. Furthermore, there is no guarantee that the upper part will be held in contact with the body of the wearer during use.

PCT/SE96/01061 describes another two-part absorbent article in which the two parts are movable in relation to one another. This known article also has limited mobility between the parts and is to a certain extent dependent on the movements of the briefs.

One way of attempting to reduce the risk of edge leakage caused by deformation of the sanitary towel during use is to provide the sanitary towel with a preshaped raised portion, what is known as a hump, which is intended to make contact with the genitals of the wearer during use of the sanitary towel. Discharged bodily fluid can in this way be caught as soon as it leaves the body of the wearer and be absorbed immediately into the article instead of running out over the surface of the latter. A raised portion also makes it easier for the wearer to position the article correctly in relation to the body. French patent publication FR-A-2 653 328 describes a sanitary towel with a hump in the form of a central, longitudinal, cylindrical raised portion.

A common way of creating a raised portion has been quite simply to build it up by arranging a greater quantity of absorption material within the area of the raised portion. As the absorption material used is in most cases what is known as cellulose fluff pulp, however, such a raised portion collapses and loses its shape when it is wetted. In order to produce a raised portion which is sufficiently large in the wet state as well, a raised portion consisting of cellulose fluff pulp must comprise so much absorption material that it is altogether too high, hard and uncomfortable to wear in the dry state.

It is also known to produce an article with a raised portion facing the wearer by positioning a shaping element on top of the absorbent core. The disadvantage is that this interferes with the liquid transport down to the absorbent, liquid-retaining absorption core and that leakage can occur because the shaping element does not have sufficient admission capacity or temporary retention capacity. The use of, for example, a foamed material in the raised portion has been proposed. However, it has proved difficult to produce a foamed structure with sufficiently open pores for good liquid admission into the latter at the same time as the material is to have such great retention capacity that liquid is not pressed out in the event of loading originating from the wearer, for example when the latter sits down.

Another example of a raised portion is described in Swedish patent 507 798. Such a raised portion has a predictable shape, both before and during use, and also keeps its shape irrespective of the movements of the wearer and of the wetting to which it is subjected. The raised portion is anatomically designed, which means that it is relatively narrow in order to project in slightly between the labia of the wearer during use without causing discomfort for the wearer.

Although such a raised portion functions well for its purpose, it has been found that when the raised portion is exposed to large quantities of bodily fluid over a relatively short period of time, there is a risk that some of the liquid will run on the outside of the raised portion and flow out past the side edges of the absorbent article. Such leakage can occur, for example, when the wearer of a sanitary towel has been sitting or lying down for a relatively long period of time and then suddenly rises. This is because, when the wearer is sitting or lying down, a relatively large quantity of menstrual fluid accumulates in the vagina of the wearer. In the event of a sudden change in body position, the entire quantity of accumulated liquid may be discharged at once. A narrow raised portion of the type described in SE 507 798 does not then have a sufficiently large surface to be capable of receiving and absorbing the entire quantity of liquid in one go, for which reason such sudden liquid flows often result in leakage.

EP 0 335 252 and EP 0 335 253 have proposed providing an absorbent article with a deformation element. The deformation element is acted on by the transverse compressive forces between the thighs of a wearer. The purpose of the deformation element is to cause a portion of the article to bulge in the direction of the body of the wearer during use. It is impossible, however, to control or predict entirely the shape the article will adopt for each individual wearer. Moreover, it is not possible to ensure contact between the body of the wearer and the surface of the article, because the degree of bulging is determined entirely by how much the article is compressed in the transverse direction.

US 4 804 380 describes an absorbent article which has a permanent three-dimensional shape. The article has one end portion of flat or concave shape and one end portion provided with a raised portion. The flat or concave end portion is intended to be positioned in front of the mons Veneris of the wearer, and the end portion comprising the raised portion is intended to fit in against the buttocks of the wearer. The three-dimensional design of the article is brought about by folding a fairly stiff absorption body. In order to make the raised portion permanent, the rear side of the article is provided with a glued surface in the end portion which is to have the raised portion. When the raised portion has been formed, it is maintained by means of the glue.

There are absorbent articles on the market which have a permanent, three-dimensional, boat-like shape and in which the outer shell consists of a moulded polymer foam.

A considerable disadvantage of permanent three-dimensional products is that it is difficult to pack a stiff three-dimensional product. Such products require a great deal of space for transport and sale, and it can be embarrassing for a wearer to carry around a sanitary towel or an incontinence pad which it is impossible to fold and therefore cannot be concealed in the hand or in the worst case will not even fit in a handbag.

EP 155 515 describes how an absorbent article, such as a sanitary towel, is provided with a bowl-shaped appearance by virtue of elastic being applied in a pretensioned state at the longitudinal side edges of the article. The use of elastic complicates manufacture, and there is a risk of the intended elastic effect being lost in connection with packing of the article or when the latter is stored in a folded packing state.

It is previously known to design plane absorbent articles which adopt a three-dimensional, essentially bowl-like shape when applied. An example of this is described in US 4 655 759. This discloses an elongate sanitary towel which consists of a layer of absorbent material, a flexible liquidtight outer layer and a liquid-permeable inner layer. The sanitary towel is provided with a pair of channels formed by stamping, the channels being located on both sides of a longitudinal centre axis and extending along a curved path over the absorption material layer. The two paths together form an hourglass-like shape positioned centrally over the towel. Before use, the sanitary towels are essentially plane but, when they are applied to the wearer, they are folded into a bowl-like shape, that is to say with liquid-stopping upright borders outside the channels. One disadvantage of this bowl-like construction is that the borders hold the central portion of the sanitary towel at a distance from the genitals of the wearer, and liquid discharged from the wearer does not flow directly into the absorbent article but can run on the surface, the risk then being obvious that liquid may find an undesirable transport path in the form of a small crease or the like and run straight out of the product in the lateral or longitudinal direction. Stamped channels in an absorption body also have the disadvantage that the liquid spread in the absorption layer is disrupted and that absorption material outside the channels is not utilized, which increases the risk of local oversaturation and attendant leakage from those parts of the absorption layer which are used.

Previously known sanitary towels and the various problems associated with them have in the main been discussed above. However, what has been said above also applies to incontinence pads. Nappies for children and adults also belong to the same problem area as far as fit in the crotch and take-up of liquid in an absorption body are concerned.

As emerged above, great efforts have been made over many years in order to attempt to solve all the problems associated with absorbent articles, such as sanitary towels. Although great improvements have been made, all the previously known solutions are associated with some disadvantages.

### DISCLOSURE OF INVENTION

By means of the present invention, an improved absorbent article of the type mentioned in the introduction has been produced. The article according to the invention is characterized mainly in that the stiffening element is in a plane state before use of the article, in that the stiffening element extends in the longitudinal direction of the article over at least part of the rear portion of the article from the crotch portion, in that the stiffening element has in the rear portion at least one elongate second through-hole which extends in the longitudinal direction of the article and along the centre line of the article, as a result of which the article is during use provided, by virtue of lateral forces arising in the rear portion of the article, with a fold along the longitudinal direction of the article along said second hole, which fold extends into the cleft between the buttocks of the wearer during use of the article and in this way stabilizes the article in position on the wearer.

An absorbent article according to the invention has a number of advantages. It is plane before use, and there are therefore no problems associated with packing, storing and transporting said article. One advantage of said design is that the article ends up in the correct place on the wearer when it is put on. The fold formed directly in front of and along the second hole holds the article in place in the longitudinal direction in the crotch of the wearer at the same time as the fold formed holds the article in place in the lateral direction by virtue of penetrating the cleft between the buttocks of the wearer.

According to an embodiment, the invention is characterized in that the second hole is pointed at its end next to the crotch portion, and in that the width of the second hole increases continuously from said end in the backward direction, as a result of which the height of the fold increases continuously in the same direction during use of the article.

According to an embodiment, the invention is characterized in that said second hole is located symmetrically and forms an angle of between 10 and 120°, preferably between 15 and 40°, at said point.

According to a suitable embodiment, the invention is characterized in that the stiffening element also extends over the crotch portion and at least part of the way in over the front portion, in that the stiffening element has a width at the transition between the crotch portion and the front portion which is adapted to the distance between the muscle tendons of the wearer on both sides of the crotch of the wearer in the groin of the latter and which is of the order of 15-45 mm, in that, in the front portion of the article, the side edges of the stiffening element diverge in the direction from the crotch portion at least part of the way in over the front portion, and in that the side edges of the stiffening element form, in the direction from the crotch area, an acute angle with a line in the longitudinal direction of the article.

An absorbent article according to the latter embodiment automatically adopts a three-dimensional bowl-like shape in an area in the front portion next to the crotch portion when the article is, at its transition between the front portion and the crotch portion, fixed in between said muscle tendons. It is known that the distance between said muscle tendons is relatively similar for all people. Fatness of course has an effect on the width between the thighs, but the width between the muscle groups is the same, and it is these which may cause an article to feel as if it chafes. The fat tissue lies on the outside of the muscles but does not contribute to any sensation of discomfort. The distance between said muscle tendons is the same irrespective of whether the wearer is slim, of normal weight or overweight. It has been found that what determines whether a wearer experiences discomfort in the form of pressure or chafing against the insides of the thighs is whether the absorbent article has a width during use which in the critical area considerably exceeds the distance between the muscle tendons in the groin portion. This distance has been found to be roughly 25-45 mm. It has been found that an article with a width which exceeds 40 mm in the critical area during use feels uncomfortable to wear to the majority of wearers. On the other hand, it is rarely experienced as being unpleasant if an absorbent article pushes down or aside fat tissue which may be present in the crotch area of the wearer.

Surprisingly, it has been found that this distance between said muscle tendons does not change throughout the lifetime of a person. Small infants therefore have a corresponding critical distance, which, according to the present invention, can be utilized for producing nappies for infants with an improved fit. The same of course applies for nappies for adults. It should be pointed out that said critical distance between the muscle tendons applies for men also, who have the same distance between said muscle tendons.

An article designed according to the latter embodiment of the invention is particularly well adapted to the anatomy of the wearer. The special geometry around the transition between the crotch portion and the front portion results in an article being anchored firmly in the groins of the wearer during use, and in this way the article is prevented from moving backwards between the legs of the wearer. This is otherwise a common problem in conventional articles because the leg movements of the wearer often shift the article backwards. This, in combination with the fold formed along the second hole, which fold, as mentioned above, holds the article in place on the wearer in both the lateral direction and the longitudinal direction, results in an article according to this embodiment staying in place well on the wearer simply by virtue of its geometry.

According to a preferred embodiment, the invention is characterized in that the stiffening element is absorbent and at the same time constitutes the absorbent element, and in that it swells during absorption while retaining its geometry in the transverse direction of the article.

It is of course possible to have a separate stiffening element behind the absorption element, seen from the side facing the wearer. A completely separate stiffening element, which has only a stiffening function, can consist of an element, made of paper or plastic for example, which is stiff in relation to the rest of the article and can be constructed from one or more material layers made of the same material or different materials. Alternatively, the stiffening area can be brought about by virtue of the article having been stiffened in this area by extra bonding agent between individual material plies. Alternatively, the article can consist of material which is permanently compressible at least in the area which is to be stiffened, suitable compression, if appropriate with heat and/or moisture being supplied, taking place during manufacture of the article to bring about the desired stiffness in the area concerned.

Depending on the selection of absorption material, it may be suitable from the point of view of function to separate the absorption element from the stiffening element. For example, a soft wadding with an open structure for rapid liquid admission and with superabsorbent material mixed in can constitute an effective absorbent material, and such a design requires a separate stiffening element. Another example in which a separate stiffening element is required is when use is made of an absorption element in the form of a foam with open pores and superabsorbent material mixed in.

However, in terms of production, it is simpler if a separate stiffening element can be eliminated.

The width of the stiffening element at the transition between the crotch portion and the front portion is of the order of 20-35 mm. It has been found that a width of 30-32 mm at said transition fits well for of the order of 80% of all wearers. According to an embodiment, the invention is characterized in that said width of the stiffening element at the transition between the front portion and the crotch portion is of the order of 25-30 mm.

The stiffening element suitably has a stiffness of in the order of 1-15 N measured according to ASTM D 4032-82. This "Circular Bend Procedure" is described in detail in EP 336 578.

According to a preferred embodiment, the invention is characterized in that the stiffening element consists of a dry-formed fibre mat with a density between 0.15 and 0.75 g/cm³ and a weight per unit area of in the order of 100-400 g/m².

A dry-formed fibre mat of this kind is described in US 5 730 737. The fibre mat produced is very stiff after forming and compression. The fibre mat can be used as it is or be mechanically softened to the desired stiffness.

A way of very accurately forming fibrous webs for use as absorption elements in absorbent articles is described in Swedish patent application 0101393-7. The fibrous webs are formed by air-laying fibres, separate air flows containing fibres being fed to a number n of different mat-forming wheels, where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method. The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

According to a suitable embodiment, the article according to the invention is characterized in that the side edges of the stiffening element, which diverge at least part of the way from the crotch portion in over the front portion of the article, are arranged so as to form an angle between a line in the longitudinal direction of the article and each of said side edges of in the order of 35-55°, preferably in the order of 45°. With this geometry in and around the transition between the crotch portion and the front portion, effective anchoring is obtained without the wearer experiencing any discomfort in the form of chafing or the like.

According to an embodiment, the article according to the invention is characterized in that the crotch portion has a length of in the order of 70-120 mm, and in that the side edges of the stiffening element diverge in the direction from the crotch portion at least part of the way from the latter in over the rear portion of the article.

Said length of the crotch portion of the article corresponds to the length of a plane portion in the crotch portion of a woman. The stiffening element according to the last embodiment is therefore anchored both at the front and at the rear at the transition between the crotch portion and the front portion and, respectively, at the transition between the crotch portion and the rear portion, as a result of which an article which is very stable, well fixed and at the same time comfortable during use is obtained.

According to an embodiment, the invention is characterized in that the article is arranged so as, by virtue of the stiffness selected for the stiffening element and by virtue of said geometry around the transition between the crotch portion and the front portion, when the article is positioned in connection with it being put on with the transition between the front portion and the crotch portion between said muscle tendons, to be fixed in between these and in this way be transformed from plane form to three-dimensional form with the front portion curved upwards in relation to the crotch portion and forming a bowl-like shape at least in an area next to the crotch portion.

Further advantageous embodiments of the article according to the invention emerge from the subsequent patent claims.

### DESCRIPTION OF FIGURES

The invention will be described in greater detail below with reference to illustrative embodiments shown in the accompanying drawings, in which:
- Figure 1: shows a plan view of an absorbent article according to a first embodiment;
- Figure 2: shows a section along the line II-II in Figure 1 but in a curved utilization state;
- Figure 3: shows an embodiment, slightly modified in relation to the embodiment according to Figure 1, of an article according to the invention in a plan view;
- Figure 4: shows a section along the line IV-IV in Figure 3;
- Figure 5: shows a plan view of a third embodiment of the article according to the invention;
- Figure 6: shows a plan view of a fourth embodiment of the article according to the invention seen from that surface of the article which receives bodily fluids;
- Figure 7: shows a plan view of the article according to Figure 6 from the opposite side;
- Figure 8: shows a section along the line VIII-VIII in Figure 6 but in a curved utilization state;
- Figure 9: shows, in perspective and in a utilization state, the article according to the fourth embodiment and also the embodiment shown in Figures 6-8;
- Figures 10-12: show plan views of three further embodiments of articles according to the invention;
- Figure 13: shows a plan view of an absorbent article according to a further embodiment;
- Figure 14: shows a plan view of an absorbent article according to a further embodiment, and
- Figure 15: shows a plan view of an absorbent article according to a further embodiment.

### MODE(S) FOR CARRYING OUT THE INVENTION

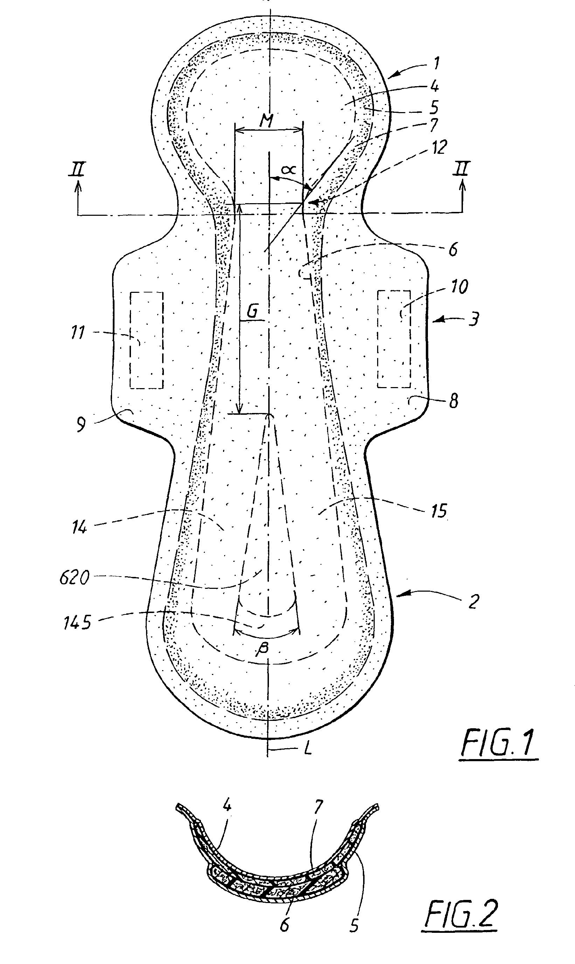

Figures 1 and 2 show an article according to the invention in the form of a sanitary towel or incontinence pad. The article is elongate with a longitudinal direction and a transverse direction. The article has a front portion 1, a rear portion 2 and a crotch portion 3 located between said portions. The article shown in Figures 1 and 2 comprises a liquid-permeable inner layer 4 which is intended to face the wearer during use of the article. The inner layer, which makes contact directly with the skin of the wearer, is suitably made from a soft, textile-like material. Examples of suitable liquid-permeable materials are various types of what are known as non-woven fabrics. Other examples of suitable materials are perforated plastic films. Net and knitted or woven textiles as well as combinations and laminates of said materials can also be used as the inner layer. Examples of inner layers for sanitary towels are laminates of various non-wovens and laminates of non-wovens and perforated plastic films. The liquid-permeable layer can also be integrated with underlying drainage or absorption layers; for example a foam plastic with open pores and with a density gradient in the depth direction can serve as a surface layer and as a drainage layer and/or absorption layer.

The absorbent article also has a liquidtight outer layer 5. This usually consists of a thin plastic layer, made of polyethylene for example. It is also possible to use a liquid-permeable material which has been treated with hydrophobing agent in order to make it liquidtight. In particular if the absorbent article is relatively large, it may be suitable for the outer layer to be breathable and also, if appropriate, vapour-permeable in addition to being liquidtight. Such layers can consist of hydrophobed non-woven fabric or of porous plastic films.

The embodiment of the absorbent article shown in Figures 1 and 2 includes an absorbent element 6 of keyhole-like shape, and a liquid-permeable insulating layer 7 which likewise has a keyhole-like shape but with a greater extent in both the longitudinal direction and the transverse direction than the absorbent element 6. The outer layer 5 and the inner layer 4 extend with edge portions outside the insulating layer around the latter and are interconnected along these edge portions to form a cover around the absorbent element 6 and the insulating layer 7. In the region of the crotch portion 3, the cover formed by the inner and outer layers extends outwards in the lateral direction to form flexible side flaps 8, 9, what are known as wings, which are intended to be arranged around the crotch portion on the briefs of the wearer in order to protect the edge portions of the briefs from soiling. The wings 8, 9 are suitably provided with adhesive coating, which has been indicated in Figure 1 by reference numbers 10, 11, on the outer layer 5, by means of which the wings can be attached around the legs of the briefs. As can be seen from Figure 2, the insulating layer 7 is located directly inside the inner layer 4 and is principally intended for rapidly admitting discharged bodily fluid into the underlying absorbent element 6 and forming a liquid-insulating layer so as to reduce what is known as back-wetting from the absorbent element 6 to the inner layer 4 making contact directly with the wearer.

The insulating layer can consist of, for example, an airlaid fibrous material of low density bonded together with bonding agent or thermofibre, which is marketed under the designation LDA (low density airlaid). The absorbent element 6 is, seen from the liquid-permeable inner layer 4, arranged under the insulating layer 7. In the illustrative embodiment shown here of the article according to the invention, this element is designed to take up and retain essentially all the bodily fluid discharged. The absorbent element 6 can be made from a material which has smaller capillaries than the insulating layer 7 located above and therefore draws liquid from the insulating layer and prevents back-wetting by liquid from the absorbent element to the insulating element and to the inner layer 4 which remains essentially dry during use of the article. Only when the absorbent element is saturated with liquid can transport take place from the absorbent element to the insulating layer.

The liquid-insulating layer 7 and the absorbent element 6 can of course be made from materials other than those indicated above. The important aspect is that the absorbent element 6 has greater liquid-affinity than the liquid-insulating layer 7 so that liquid is transported from the insulating layer to the absorbent element but not vice versa.

The liquid-insulating layer can consist of, for example, what is known as a multibond non-woven, that is to say a non-woven fabric in which fibres are bonded by both bonding agent and melt bonds. This can also contain fibres or particles made of a slow-acting superabsorbent material and/or an odour-inhibiting superabsorbent material.

In the illustrative embodiment shown, the absorbent element 6 is also intended to serve as a stiffening element and is to this end designed so as to be stiff in order as far as possible to avoid the absorbent article being compressed in an uncontrolled manner when squeezing forces in the lateral direction occur, generated by the thighs of the wearer in the crotch area. The absorbent stiffening element has a size, shape and stiffness which result in the article, throughout its time of use, retaining a predetermined shape and moreover being retained in the intended position on the wearer.

The expression stiffening area means that an area has been reinforced in some way in order that this area is stiffer than the rest of the article. This reinforcement can consist of a separate reinforcing element as referred to above which, as in the embodiment according to Figures 1 and 2, also serves as an absorbent element, or a completely separate stiffening element which has only a stiffening function and can consist of an element, made of paper or plastic for example, which is stiff in relation to the rest of the article and can be constructed from one or more material layers made of the same material or different materials. Alternatively, the stiffening area can be brought about by virtue of the article having been stiffened in this area by extra bonding agent between individual material plies. Alternatively, the article can consist of material which is permanently compressible at least in the area which is to be stiffened, suitable compression taking place during manufacture of the article to bring about the desired stiffness in the area concerned. The latter illustrative embodiment is described in greater detail below.

In the description below, the expressions stiffening area and stiffening element will be used interchangeably, the most suitable expression being selected in order to clarify what is meant at the point concerned in the text.

As can be seen from Figure 1, the absorbent stiffening element 6 extends over the front portion, the entire crotch portion 3 and a considerable part of the rear portion 2.

At the transition 12 between the crotch portion 3 and the front portion 1, the stiffening element 6 has, in the illustrative embodiment shown here, a width M which is adapted to the distance between two particular muscle tendons on both sides of the crotch of the wearer directly in front of the groins. These muscle tendons form part of the muscle group which originates on the inside of the pelvic diaphragm and has its attachment along the thigh. This muscle group consists of the adductor brevis, adductor longus, gracilis and adductor magnus muscles. As mentioned above, it is known that this distance between said muscle tendons is relatively similar for all people. This dimension is of the order of 25-45 mm. Research has shown that 80% of all women have a dimension of roughly 30-32 mm between said muscle tendons. When said width M essentially corresponds to the distance between said muscle tendons on the wearer, the article will be anchored firmly during use with the transition portion between the muscle tendons and be retained in this position. The two side edges of the front portion diverge in the forward direction on the article from said transition area 12. In this way, the article is prevented from moving backwards between the legs of the wearer. This is a common problem in conventional sanitary towels because the leg movements of the wearer often shift the sanitary towel backwards.

In Figure 1, an angle between a line in the longitudinal direction of the article and each of said side edges has been designated by α. In the case of a large angle α, for example close to 90°, the edges of the front portion may chafe against the groins and legs of the wearer and in this way cause discomfort for the wearer. The smaller the angle α, the greater the risk that the article will slide backwards in between the legs of the wearer. In the case of an angle of less than 30°, this risk is unacceptably high. An angle of 35-45° provides the best balance between secure positioning and comfort. An angle of roughly 45° has been found to be especially favourable.

An absorbent article, such as a sanitary towel, according to the invention is designed with a crotch length adapted to the anatomy of the wearer. In a sanitary towel according to the invention, use has been made of the fact that the great majority of women have a crotch length of in the order of 80-100 mm. The stiffening element 6 has therefore been designed with a corresponding crotch length G of in the order of 70-120 mm, that is to say the distance from the transition area 12 to the start of the rear portion.

Along the crotch, where the body shape of the wearer is essentially plane, the sanitary towel according to the invention is designed so as in the dry state to be relatively stiff in the lateral direction, that is to say it is sufficiently stiff not to be deformed in an uncontrolled manner in the lateral direction and form creases. As the stiffening element 6 in the embodiment described here also constitutes the major part of the absorption capacity of the sanitary towel, it is essential for it to be possible to utilize available space between the legs of the wearer in the crotch. The width of the sanitary towel in the crotch area is, with regard to the stiffening element, limited at the front by said distance between said muscle tendons directly in front of the groins of the wearer. In the backward direction from said transition area to the end of the crotch portion, the width of the stiffening element 6 and thus the absorbent element can increase continuously to of the order of 1.5 times the width in the transition area 12 between the crotch portion and the front portion without any risk of the stiffening element chafing the wearer in the crotch.

The abovementioned geometrical design of the area in and around the transition area 12, that is to say the size of the angle α and the width M, and also the selected crotch length G on the stiffening element for the article according to the invention, affords a very good anatomical adaptation of the stiffening element, which gives the article a good fit and stability in the fitted position on the wearer. This is of particularly great importance for the functioning of the article, not least because the wetting point can, on account of the body position of the genitals of the wearer in the longitudinal direction of the crotch area, vary for different wearers. As the available space around the wetting point is very limited in width and length, optimum positioning and anchoring in this position of the stiffening absorbent element is necessary. This is achieved by means of said distances M and G selected and said angle α selected.

The anchoring effect is achieved at said muscle tendons even when the width M on the article is slightly less than the distance between said muscle tendons directly in front of the groins. The two edge portions of the front portion diverge in the forward direction, and the article can slide backwards slightly until the edge portions are anchored firmly between said muscle tendons. The distance M on the article is suitably of the order of 15-35 mm and preferably 25-30 mm. The latter distance fits most wearers. If the distance exceeds roughly 35 mm, articles may therefore feel uncomfortable to some wearers. A distance in excess of 45 mm is unsuitable because such articles cause discomfort in the form of chafing for most wearers.

The stiffening element 6 and therefore the absorption element also extend part of the way in over the rear portion 2 of the article. In the rear portion, the stiffening element has an elongate second through-hole 620, as a result of which a weakening is formed so that, during use, the article can fold along a longitudinal line L in the hole 620 and as a result of which the stiffening element forms legs 14 and 15 which are located on both sides of the hole 620 and are more flexible than the wider crotch portion. The legs 14 and 15 can be made slightly vertically movable in relation to one another by virtue of the width selected for the hole. The hole 620 is very important for the adaptation and flexibility of the article in relation to the body. The fold formed along the hole during use of the article can penetrate the cleft between the buttocks of the wearer and in this way provides very good protection against leakage via the cleft between the buttocks, which type of leakage usually occurs during the use of conventional products when the wearer is lying on her back. The fold formed, which projects into the cleft between the buttocks of the wearer, also results in the article being stabilized in position on the wearer and remaining in the intended position during body movements, for example when the wearer is walking. The article is held in place on the wearer in both the longitudinal direction and the transverse direction by the fold formed at the hole 620. The two legs 14 and 15 are interconnected at the bottom at 145. This connection gives the stiffening element 6 very good stability in the rear portion and provides the article with the necessary firmness in this area.

In the illustrative embodiment shown in Figure 1, the hole 620 is wedge-shaped and located symmetrically in relation to the longitudinal symmetry line L of the article and also forms an angle β of in the order of 20°. This angle can vary within wide limits but of course depends on the design of the rear portion 2. The angle β can vary between of the order of 15° and 40°.

The hole 620 is pointed at its end next to the crotch portion 3, and, in the illustrative embodiment shown, the width of the hole increases continuously from said end in the backward direction. As a result of this, the height of the fold formed will increase continuously in the same direction during use of the article, and this increasing height of the fold prevents the article being displaced forwards.

In the illustrative embodiment shown, the stiffening element 6 also serves as the main absorption element of the article and has very great liquid-spreading capacity for rapid spreading of bodily fluid received from the wearer in the narrow crotch area directly in front of the genitals of the wearer over the absorbent portions of the whole article, that is to say over the entire stiffening and also liquid-absorbing element 6. This stiffening absorbent element is designed so as to swell in the depth direction during absorption and on the whole retain its geometry in the transverse direction of the article, which results in the stiffening element retaining its fit and secure positioning in relation to the body of the wearer throughout use of the article. The absorbent stiffening element 6 has great swelling capacity in the depth direction and attendant great absorption capacity.

According to a suitable embodiment, the stiffening absorbent element 6 consists of a dry-formed fibre mat with a density between 0.15 and 0.75 g/cm³ and a weight per unit area of in the order of 100-400 g/m². A dry-formed fibrous mass in the form of a fibre mat is described in US 5 730 737. The fibre mat produced is very stiff after forming and compression. The fibre mat can be used as it is or be mechanically softened to the desired stiffness. A way of very accurately forming fibrous webs for use as absorption elements in absorbent articles is described in Swedish patent application 0101393-7. The fibrous webs are formed by air-laying fibres, separate air flows containing fibres being fed to a number n of different mat-forming wheels, where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method.

The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

The fibre mat for forming the stiffening absorbent element can consist of a mixture of cellulose fibres and viscose fibres, the presence of the latter giving the fibre mat a greater wet strength than a fibre mat made of only cellulose fibres. The fibre mat for forming the stiffening absorbent element can also contain synthetic melt fibres, by means of which the strength of the fibre mat can be increased by heat treatment to melt said synthetic melt fibres.

The absorbent stiffening elements can also be formed from foamed material.

A further example of stiffening absorbent material is a laminate in the form of one or more plies of tissue and superabsorbent material (SAPs). The material or combination of different materials serving as an absorbent element and also, if appropriate, as a stiffening element can contain SAPs in the form of fibres, particles or foam.

The selection of compression pattern also makes it possible to vary the extensibility of the fibre mat. The dry-formed fibre mat can be provided with the desired reduced stiffness and the desired extensibility by virtue of the degree of compression selected and the compression pattern selected.

Furthermore, it is possible to pattern-compress only specific zones for the purpose of providing only these zones with an extensibility and stiffness which are different from the rest of the stiffening absorption element. In the same way, the stiffening absorption element can be compressed over its entire extent but with different patterns in different zones. By virtue of the presence of a stiffening absorption element which can in a simple manner, by virtue of the pattern compression selected, be provided with the desired stiffness and the desired extension in different zones, and in which the stiffness and extension properties can be selected essentially freely in these zones, the present invention has brought about a new and previously unknown way of controlling and guiding the shaping of an absorbent article intended for taking up bodily fluids.

As mentioned above, the stiffening absorbent element 6 has great swelling capacity in the depth direction, which has been achieved by great compression of the fibre mat in connection with its production. In the dry state, the material formed, such as the fibre mat, is hard-compressed and stiff, which affords the shaped and anatomically adapted absorption element very good stability in the fitted position on the wearer and very great spreading capacity, as a result of which the total absorption capacity of the absorption element can be optimally utilized and leakage caused by local oversaturation can to a great extent be eliminated. During absorption of liquid, the absorption body swells mainly in the depth direction but the absorption element does of course swell slightly in other directions as well. When the anatomically adapted stiffening absorption element swells, further improved anatomical adaptation is in fact achieved, which contributes to the stability and flexibility of the article in relation to the body shape of the wearer when the stiffness of the absorption element decreases during absorption and attendant swelling.

So as to function in the desired manner, the stiffening element has a stiffness in the dry state of in the order of 1-15 N measured according to ASTM D 4032-82. This "Circular Bend Procedure" is described in detail in EP 336 578.

The stiffening absorbent element can also consist of a laminate of a number of non-woven fabric layers or tissue layers which are fixed to one another for increased stiffness and which can have highly absorbent particles between individual plies. The individual plies can be fixed to one another by a bonding agent, such as adhesive or melt fibres. The highly absorbent particles can also contribute to bonding. The stiffness is controlled by virtue of the selection of the number of plies and the quantity of bonding agent included and the selection of highly absorbent material and how the adhesive capacity thereof is utilized.

A stiffening absorbent element of this type can also be provided with different stiffness and different extensibility in different zones of the extent of the element. These properties can in this case as well be controlled by means of compression patterns. This compression can be combined with the supply of heat, which supply can vary in different zones. Furthermore, bonding agent can be applied in different patterns to control the shaping of the stiffening absorption element during use. A varying supply of moisture in different areas in connection with compression is another parameter for controlling the shaping of the article during use.

Another example of the construction of a unit serving as both absorption element and stiffening element is a number of layers of LDA, that is to say layers of the same type as in the drainage and insulating layer 7. However, the layers of LDA in the stiffening absorption element are bonded both within and between individual layers. This bonding is brought about by hard-compression of the LDA layers and suitably by using both melt fibres and latex, what is known as the multibond technique. In this design as well, stiffness and extensibility can be controlled by compression pattern selection and also by variation of the heat supply in different zones.

Further material examples are mixtures of LDA and HDA (high density airlaid) if appropriate in combination with other material layers, such as tissue.

Pattern compression can be used in all the material examples described above, and it is then possible to achieve, for example, hinge effects along compression lines or compression zones.

Pattern formation can take place in connection with compression of the stiffening absorption element. Alternatively, pattern compression can take place in a separate step after smooth compression. Use can be made of, for example, a web of material made in one of the ways described above and smooth-compressed as the starting material for the stiffening absorption element, which is pattern-compressed in the desired manner and depending on the type and size of article to be manufactured. After pattern-compression, individual products are cut out. Pattern-compression and cutting-out of separate stiffening absorption elements can take place in a single step in a combined cutting and pattern-compression unit.

As described above, the stiffening element can also constitute the main absorption element of the article. This is suitable from the point of view of production because there are fewer elements to handle than if, for example, the stiffening element and the absorption element constitute separate elements.

The invention also comprises designs in which the stiffening element is separate from the main absorption element of the article. The stiffening element can then be absorbent or non-absorbent. The main purpose in such a design is to constitute a stiffening shaping element.

In addition to the interpretation of the term stiffening element as constituting a completely separate element or constituting both the main absorption element and the stiffening element of the article, the term can also embrace the interpretation that all the material plies, bonding agents etc. included in the article in the area of the desired stiffening together form the desired stiffening element. In such a design, the expression stiffening area can also suitably be used instead of stiffening element. Absorption element and stiffening area can be made of one and the same material, for example a foamed material or a body constructed from fibres, the stiffening area being bonded in compressed form.

For example, a unit serving as a stiffening element and also as an absorption element, with the M and G dimensions indicated above and with the geometry described above but with stiffness which is in itself inadequate, is included in the invention if the necessary stiffness is obtained by being bonded together with other material plies in the area of the stiffening element.

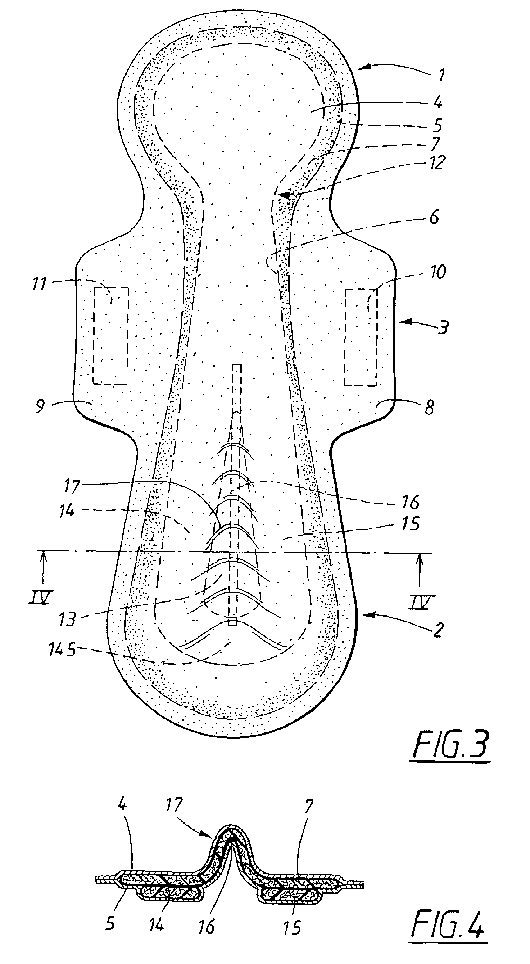

The embodiment shown in Figures 3 and 4 differs from the embodiment shown in Figures 1 and 2 only in that an elastic means 16 is arranged in a pretensioned state in the longitudinal direction of the article and centrally along the rear portion 2 of the article. The same reference numbers have been used in Figures 3 and 4 as in the embodiment according to Figures 1 and 2.

The elastic means 16 is arranged centrally in the hole 620 and extends in the rear portion slightly beyond the ends of the legs 14 and 15 in under the portion 145 connecting the legs and in the other direction part of the way in over the crotch portion. The elastic means is arranged on the inside or on the outside of the liquidtight outer layer and is connected to the latter and/or other layers forming part of the article. The extent of the elastic means 16 is not critical but can vary somewhat in relation to the illustrative embodiment shown in Figure 3. One purpose of the elastic means 16 is, during use of the article, to draw adjacent material portions together and contribute to curving the article in the upward direction towards the body of the wearer for better contact with the body. Another purpose is also to initiate and form the fold 17 which, during use of the article, is intended to penetrate part of the way into the cleft between the buttocks of the wearer, stabilize the article in position on the wearer and also prevent leakage of bodily fluid backwards along the cleft between the buttocks, which leakage can otherwise occur when the wearer is lying on her back.

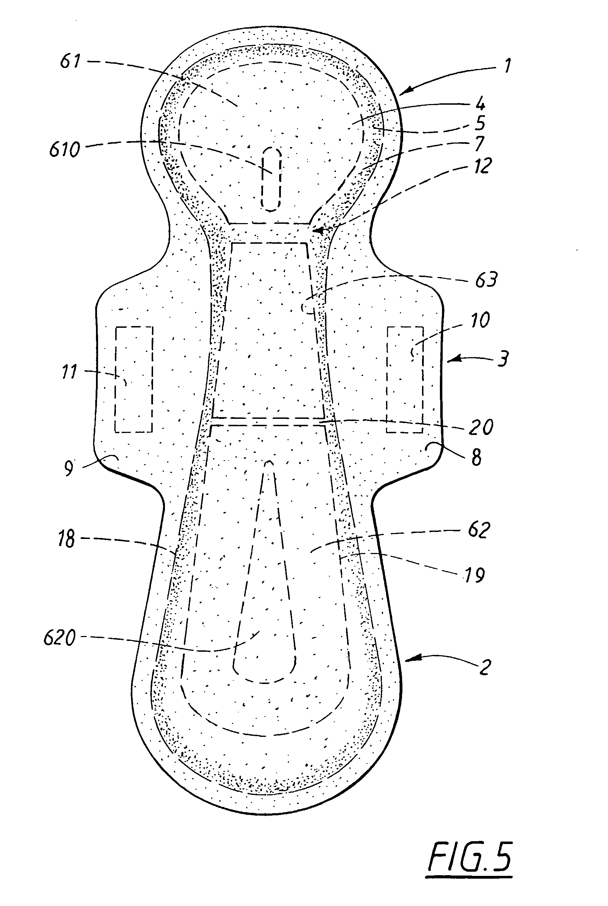

In the embodiment shown in Figure 5, the components which correspond to similar parts in the embodiments according to Figures 1-4 have been provided with the same reference numbers. The embodiment according to Figure 5 includes a stiffening element which is designated as a whole by reference number 6 and consists of a first stiffening part element 61 in the front portion 1 and a second part element 62, separate from the first part element, in the rear portion. Arranged between the two part elements 61, 62 is an absorbent crotch element 63. In the illustrative embodiment shown, the absorbent crotch element 63 is designed to be less stiff than the stiffening part elements 61, 62 in order as far as possible to avoid the absorbent crotch element giving rise to chafing when squeezing forces in the lateral direction occur, generated by the thighs of the wearer in the crotch area. The absorbent crotch element 63 is retained in the intended position by means of the stiffening part elements 61, 62.

At the transition 12 between the crotch portion 3 and the front portion 1, the stiffening part element 61 has a width M which, as in the embodiments described above, is adapted to the distance between said muscle tendons on both sides of the crotch of the wearer directly in front of the groins. The two side edges of the front stiffening part element 61 diverge in the forward direction on the article from said transition area 12. In this way, the article is prevented from moving backwards between the legs of the wearer when the wearer is walking. By virtue of the first stiffening part element 61 being separate from the absorbent crotch element at the transition 12, a certain turning of the first part element 61 in relation to the crotch element 63 and also in relation to the second part element 62 is permitted, which makes increased mobility possible for the wearer without the risk of annoying chafing caused by the stiffening part elements 61, 62.

In the embodiment shown in Figure 5, the first stiffening part element 61 has been provided with an elongate first through-hole 610, and the second stiffening part element has an elongate second through-hole 620 with the same function as the elongate second hole in the embodiments according to Figures 1-4. The stiffening part elements forming part of the embodiment according to Figure 5 can be made of the same material as described above in connection with the description of the stiffening element 6 in the embodiments according to Figures 1-4.

As can be seen from Figure 5, the first through-hole 610 arranged in the first part element is oblong and extends along the longitudinal direction of the article and along its centre line. The purpose of the first hole 610 is to facilitate curvature of the first part element and to make possible resilient compression thereof in the lateral direction when lateral forces against the side edges of the first part element arise.

The outer side edges 18, 19 of the second part element diverge in the direction from the crotch area. The purpose of the edge sides 18, 19 of the second stiffening part element 62 diverging in the backward direction on the rear portion 2 is that the article, in addition to being anchored firmly at the transition 12 between the front portion and the crotch portion, will also be anchored at the rear in the transition area between the crotch portion 3 and the rear portion 2, as a result of which the article is very stable and well fixed on the wearer during use at the same time as it feels comfortable for the wearer by virtue of its anatomical adaptation in terms of shape, size and geometry. For a good anchoring function, the angle between the longitudinal direction of the article and each outer edge side 18, 19 should not be less than roughly 30°. Furthermore, so as not to feel uncomfortable, the angle should not exceed roughly 60°.

The distance G between the transition area 12 and a transition area 20 between the crotch element and the second stiffening part element 62 is adapted to the crotch length of a wearer and, as mentioned above in connection with the embodiments according to Figures 1-4, this distance G is suitably of the order of 70-120 mm. As mentioned above, the essentially plane area of the crotch of women directly in front of the genitals has a length of in the order of 80-100 mm, that is to say all women are essentially the same size in this plane area. It has been found that a crotch dimension G on the article of in the order of 70-120 mm functions well for most wearers. The larger the angles between the side edges of the first part element and the longitudinal direction of the article and between the outer edge sides 18, 19 and the longitudinal direction of the article, and the stiffer the stiffening element, the more important it is that the crotch dimension on the article corresponds to the length of the plane crotch portion of the intended wearer directly in front of her genitals if the article is not to feel uncomfortable.

It may therefore be conceivable to have a range of sizes of the article according to the invention depending on the selection of stiffness and said angles, so that different wearers can find a suitable size with regard to dimensions and angles. This of course applies to all the embodiments of the invention described here but is particularly important when the article is intended to be anchored both at the front and at the rear. The requirement for size adaptation also increases for all the embodiments the stiffer the absorbent element is.

The second stiffening part element 62 in the rear portion 2 of the article is provided with an elongate second hole 620. As in the other illustrative embodiments described above, this hole is wedge-shaped and has the same function, that is to say it is to give rise to a fold 17 during use of the article, which fold stabilizes the article in the fitted position on the wearer in both the lateral direction and the longitudinal direction.

As mentioned above in connection with the other illustrative embodiments, the size of the hole 620 also influences the height of the fold 17.

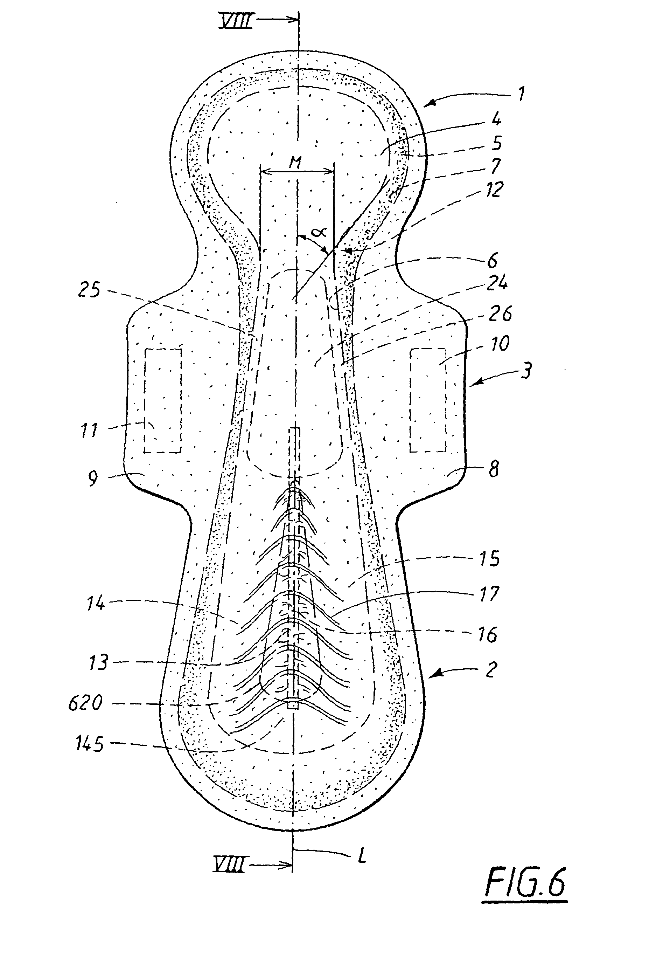

Figures 6-9 show a suitable embodiment of an article according to the invention. This embodiment corresponds in many respects to the embodiments according to Figures 1-4, and those parts corresponding to the same parts in the embodiments described above have been provided with the same reference numbers in the drawing.

A way of reducing further the risk of edge leakage caused by the sanitary towel being deformed during use, in addition to the arrangement of the stiffening element 6, is to provide the sanitary towel with a raised portion, what is known as a hump, which raised portion has been designated by reference number 240. The raised portion or hump is intended to make contact with the genitals of the wearer during use of the sanitary towel. Discharged bodily fluid can in this way be caught as soon as it leaves the body of the wearer and be absorbed immediately into the article instead of running out over the surface of the latter.

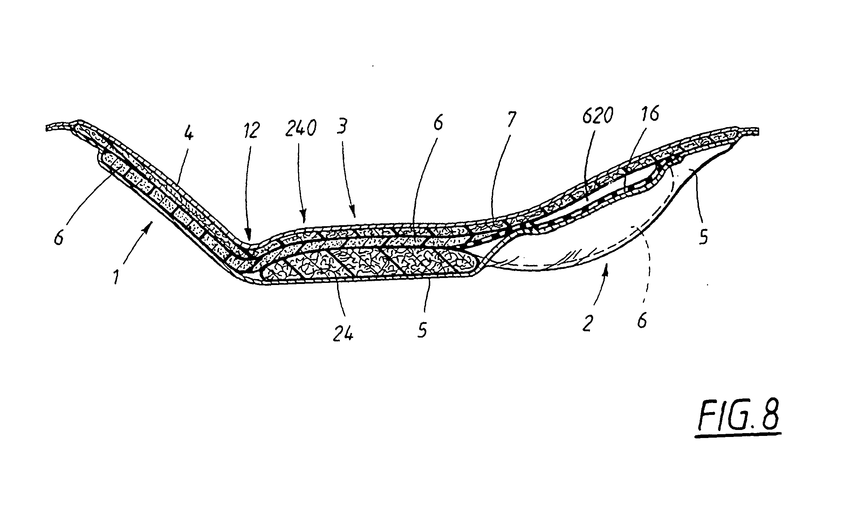

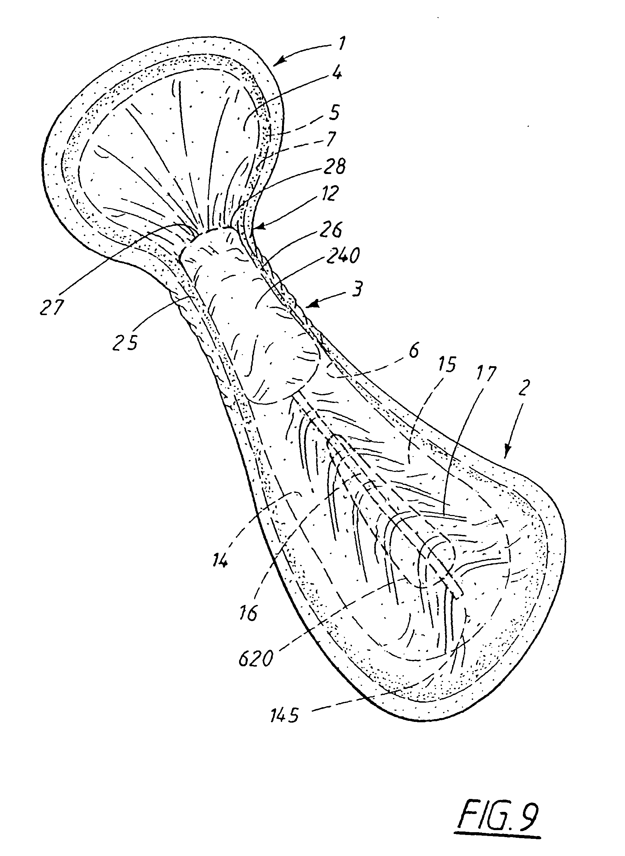

In the embodiment shown in Figures 6-9, the hump is brought about by a hump-forming element 24 which, as can be seen most clearly from Figure 8, is arranged below the stiffening element 6 inside the liquid-impermeable outer layer 5. The positioning of the hump-forming element results in a number of advantages. Admission of bodily fluid is not interfered with by hump material in direct proximity to the genitals of the wearer, but the parts located closest to the genitals of the wearer can be optimized with regard to admission and absorption capacity. The positioning selected for the hump-forming element below the stiffening element 6 in combination with the positioning along the crotch portion of the article also results in the positive effect that the article curves and shapes itself in the desired manner when fitted on the wearer. At the transition 12 between the crotch portion 3 and the front portion, as can be seen from Figure 9, a point of inflexion 27 is formed, in front of which, that is to say in the front portion of the article, the article is concave at least over a portion next to said transition 12. Behind said point of inflexion, that is to say along the crotch portion of the article, the article is, in the area directly in front of the hump-forming element 24, convex, that is to say the stiffening element 6 is curved in this area, upwards in the crotch portion 3, as can be seen most clearly from Figures 8 and 9. In addition to bringing about the raised portion 240 on the front side of the article, the hump-forming element makes it possible to guide the stiffening element in the desired direction of curvature at different points of the extent of the stiffening element.

The hump-forming element 24 consists of, for example, a non-absorbent synthetic wadding which has resilient properties. Such a hump-forming element retains its shape and function even when the material is in a wet state.

The hump-forming element can also consist of a foamed material, for example polyurethane foam or the like. If appropriate, the hump-forming element can be provided with superabsorbent material, in the form of particles or fibres, which material expands greatly during liquid absorption and expands the hump formed by the hump-forming element.
As the hump-forming material is, in the embodiment shown, located below the absorbent element 6, which also serves as the stiffening element, the hump-forming material can be liquid-absorbing. In such a design, it is suitable to select a material which has larger capillaries than the absorption element has, so that liquid can be transported to the hump-forming material only when the absorption element is saturated with liquid. A hump-forming absorbent fibrous layer which has resilient properties only in the dry state can therefore also be used in such a construction because the material is essentially dry until the absorption element itself is saturated with liquid. The positioning of the hump-forming element 24 below both the stiffening and the absorbent element therefore affords a number of important advantages.

The element forming the raised portion 240 has an elongate shape and extends over the entire crotch portion in the illustrative embodiment shown. The length of the raised portion can vary between roughly 20 mm and 120 mm.

The element 24 forming the raised portion is narrower than the rest of the article in the crotch area. In this way, it is made possible for laterally surrounding portions 25, 26 of the rest of the article to shape themselves around the element 24 forming the raised portion. The material forming the raised portion is suitably at least twice as thick as the surrounding areas 25, 26.

In Figure 8, the article has been shown in curved, three-dimensional form for the sake of clarity. An absorbent article of the type described here is of course always three-dimensional in the conventional sense, that is to say it has length, width and thickness.

In this context, however, the term three-dimensional means that the article must be curved in some way to adapt to the body shape of the wearer.

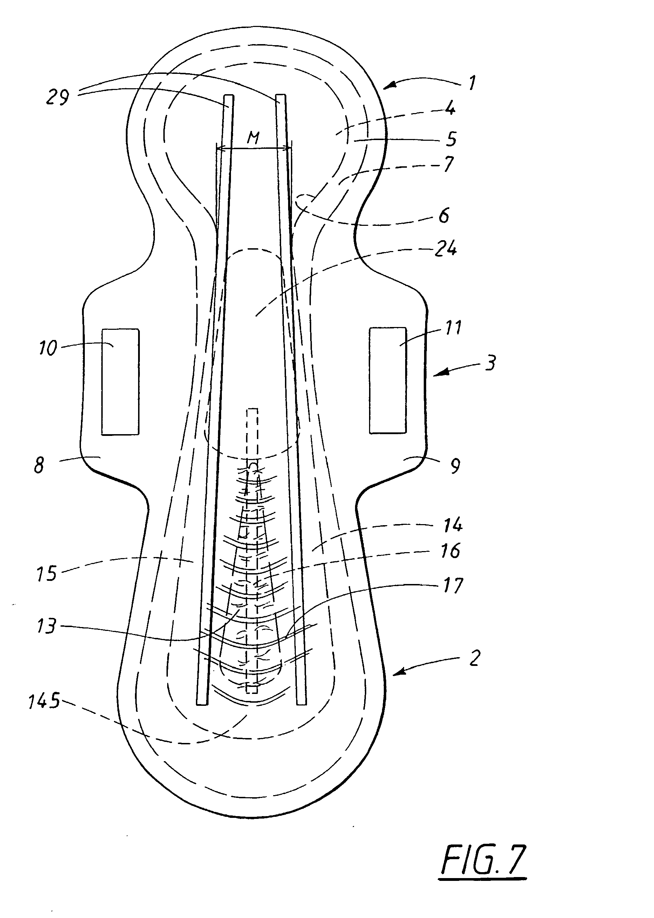

In this context, the term plane form means that the article is essentially plane. The article shown in Figures 6 and 7 is essentially planiform according to this definition in spite of the fact that the elastic means draws the material layers together in the second hole 620 between the legs 14, 15.

Articles in plane form according to Figures 6 and 7 can be packed simply, for example in stacks in a box or bag and yet, when put on, be made to adopt an anatomically adapted three-dimensional shape, as shown in Figures 8 and 9, without any measures whatsoever.

By virtue of its special design with the dimension of the distance M between said muscle tendons, the hump-shaped element 24, the action of the elastic means 16 and the stiffness and geometric shape of the stiffening element 6, the article is anatomically adapted and predestined to adopt during handling a three-dimensional shape according to Figures 8 and 9 adapted to the body shape of the wearer.

In the illustrative embodiment shown, the stiffening and also absorbent element 6 has the same stiffness properties over its entire extent. As a result, uncontrolled creases, which could give rise to uncontrolled and unintentional liquid flow, do not normally arise over the extent of the stiffening element. At the transition 12 between the crotch portion 3 and the front portion 1, curvature is initiated because the article as a whole changes its flexural resistance here, on the one hand on account of the hump-forming element having its end directly in front of this transition and on the other hand because the stiffening element is narrowest here with a dimension M adapted to the distance between said muscle tendons on the wearer. At this transition 12, a point of inflexion 27 is formed, in front of which the article is concave and bowl-shaped, whereas it adopts a convex shape behind this point of inflexion 27. In the embodiment according to Figure 9, the hump-forming element is rounded at the front along a line 28. In this way, the stiffening element is caused by this rounded line to adopt an evenly rounded bowl shape in the front portion, as can be seen from Figure 9.

In the transition area 20 between the crotch portion 3 and the rear portion 2 as well, the hump-forming element 24, which in the embodiment shown extends as far as said transition area 20, is rounded at its rear end. As a result, no undesirable creases arise, but the transition between the convex crotch portion and the two side portions of the rear portion 2 sloping downwards around the fold 17 formed by the elastic means 16 is even and smooth without undesirable creases.

The raised portion 240 formed by the hump-forming element 24 also has the advantage that the fold extending into the cleft between the buttocks of the wearer does not extend in too abruptly or too far and give rise to chafing. In this respect also, the hump provides a soft transition in the transition area between the crotch portion and the rear portion.

In all the embodiments described above, it is suitable for the article to be provided with a pressure-sensitive adhesive on the outside of its liquid-impermeable outer layer 5. This has been indicated in Figure 7 by adhesive strands 29 which, before use of the article, are covered in a conventional manner by a cover strip 30 treated with release agent. Although the article according to the invention is anatomically adapted, it is suitable, for reliable secure positioning, to have a pressure-sensitive adhesive on the liquid-impermeable outside of the article for interaction with the briefs of the wearer, which contributes to keeping the article in the intended position on the wearer. The selection of suitable attachment, that is to say whether and to what extent pressure-sensitive adhesive for attachment to the briefs of the wearer is to be used, is guided by the selection of the stiffness of the stiffening element included.

According to an embodiment (not shown in the drawing), the article can be attached to or interact with the body of the wearer by means of adhesive or friction coating. The friction means or adhesive can be the only means of attachment, but it can also be used in combination with pressure-sensitive adhesive intended for attachment to the briefs of the wearer.

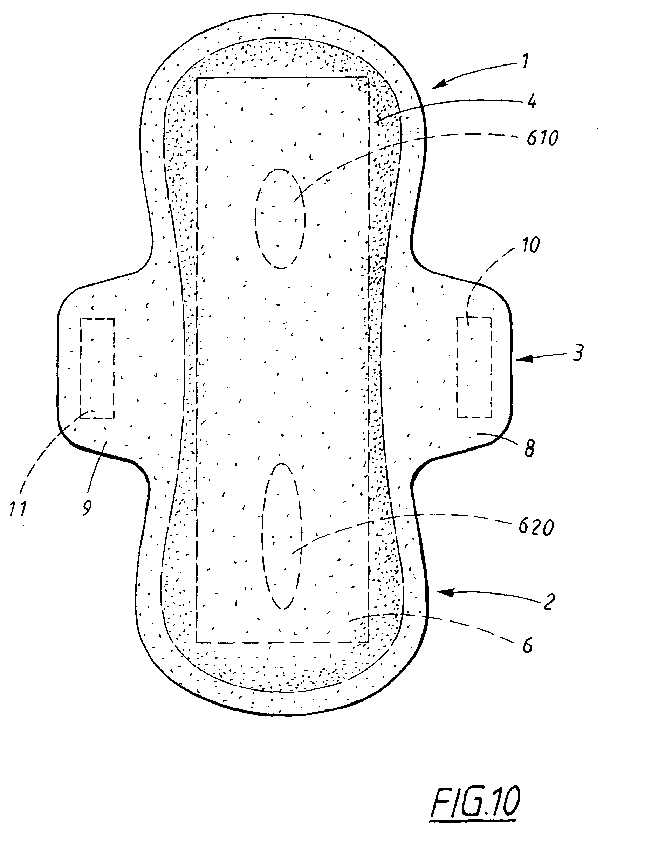

Figure 10 shows an embodiment which is modified slightly in relation to the embodiments described above. Those parts in the article according to Figure 10 corresponding to similar components in the embodiments according to Figures 1-9 have been provided with the same reference numbers.

The stiffening element 6 in the embodiment according to Figure 10 is rectangular and has an elongate through-hole 610 at the transition 12 between the front portion 1 and the crotch portion 3. This hole is intended to make possible pressing-together of the stiffening element 6 in the lateral direction, which pressing-together is at a maximum where the hole is widest when pressing-together takes place. During pressing-together, curvature of the article also takes place, the outer portions of the stiffening element 6 at the front being curved upwards towards the body of the wearer. After pressing-together, the stiffening element 6 has a width M during use which is adapted to the distance between said muscle tendons on both sides of the crotch of the wearer directly in front of the groins. The desired width M is controlled by the stiffness and width of the stiffening element and also by the width of the hole at the point concerned.

As can be seen from the above, it is the first through-hole 610 which makes it possible to press the article together at the transition 12. By virtue of the selection of the stiffness of the stiffening element, the shape and size of the hole and also the material width of the stiffening element in the area directly in front of the hole, the desired resilience can be obtained when the article is pressed together. The result of this is that an article of a certain size can fit wearers with slightly different widths between said muscle tendons, that is to say the hole 610 can be pressed entirely or partly together during use of the article.

After an article according to Figure 10 has been pressed together during use, the two side edges of the front portion diverge in the forward direction on the article from said transition area 12. In this way, the article is prevented from moving backwards between the legs of the wearer when leg movements take place. In the case of the embodiment according to Figure 10, essentially the same shape of the front portion of the article and the transition area is therefore obtained during use of the article as in the case of the articles preshaped with anatomically adapted outer contours according to the embodiments described in connection with Figures 1-9.

In the rear portion, the stiffening element 6 has an elongate through-hole 620 which extends, backwards in the longitudinal direction of the article and centred along the centre line of the article, from the transition area 20 between the crotch portion 3 and the rear portion. During use, the article is thus, as in embodiments described above, provided with a fold along the longitudinal direction of the article along said second hole 620. During use of the article, this fold extends into the cleft between the buttocks of the wearer and stabilizes the article in position on the wearer.

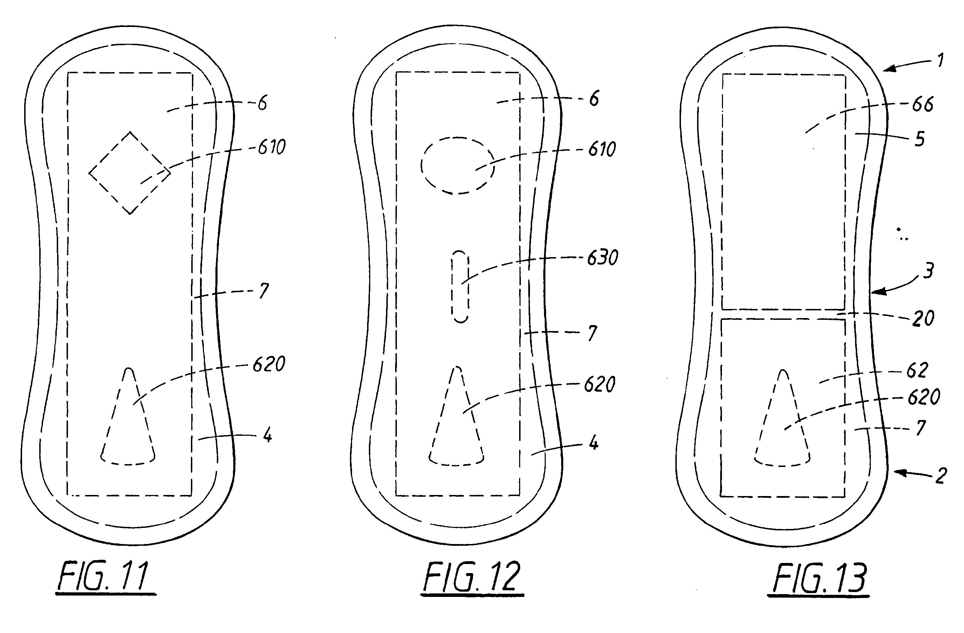

Figures 11 and 12 show further examples of the stiffening element for an absorbent article according to the invention. In the embodiments according to Figures 11 and 12, those parts corresponding to similar components in illustrative embodiments described above have been provided with the same reference numbers.

In the embodiment according to Figure 11, the stiffening element 6 extends over the front portion 1, the crotch portion 3 and the rear portion 2 of the article. In the front portion, the stiffening element is provided with a diamond-shaped through-hole 610 which is widest in the transition area 12 where the article is intended to be anchored between said muscle tendons during use of the article. By means of the triangular hole, continuously decreasing curvature of the stiffening element is obtained from said transition 12 in the direction towards the front end of the article.

In the rear portion 2 of the article, as in the embodiments according to Figures 1-10, a longitudinal hole 620 is arranged. This has the same function as described in said embodiments and, during use of the article, gives rise to a fold, the height of which increases in the backward direction. During use of the article, the fold stabilizes it in the lateral direction as described above.

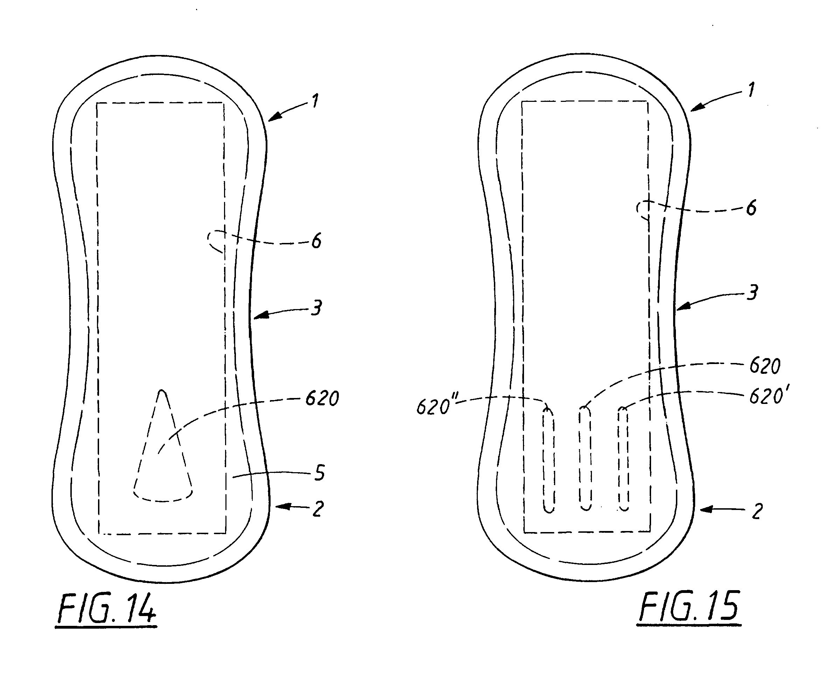

Figure 12 shows an article, the stiffening element 6 of which, as in the embodiment according to Figure 11, extends over the front portion 1, the crotch portion 3 and the rear portion 2 of the article. In this case, the stiffening element has an elliptical hole 610 directly in front of the transition 12, which hole is intended, during use of the article, to make possible squeezing-together of the stiffening element at the transition 12 to bring about a narrowing in this area to a width M which corresponds to the distance between said muscle tendons on the wearer. A longitudinal hole 630 is arranged in the crotch area of the article. The purpose of the hole 630 is to provide the stiffening element with resilient properties in the crotch area for optimum adaptation of the width of the stiffening element in the crotch area to the body shape of the wearer in this area. The hole 620 has the same function as the corresponding hole in the embodiment according to Figure 15 and is therefore not described further.

Figure 13 shows an embodiment which differs from embodiments described above mainly in that the stiffening element consists of only a part element 62 in the rear portion 2 of the article. The part element 62 is rectangular and has an elongate hole 620 of the same kind and with the same function as described in illustrative embodiments described above.
There is no stiffening element in the front portion 1 or in the crotch portion 3. Arranged here instead is an elongate absorption element 66 which is less stiff than the part element 62.

During use of an article according to Figure 13, the article is held in the intended position on the wearer by means of the stiffening part element, the fold of which formed along the hole 620 stabilizes the article in the lateral direction and in the longitudinal direction on the wearer. The fold projects into the cleft between the buttocks of the wearer and therefore stabilizes the article in the lateral direction. Moreover, the height of the fold increases continuously in the backward direction corresponding to the continuously increasing width of the hole in the same direction, which results in an article fitted on the wearer being stabilized against displacement in the longitudinal direction of the article.

The flexural rigidity of the article increases after it has been fitted on the wearer, which, as mentioned above, is on account of the rear portion of the article being more stable. The fold formed along the hole 620 will, during use of the article, penetrate part of the way into the cleft between the buttocks of the wearer and in this way in addition contribute to the article staying in place in the lateral direction at the same time as catching any bodily fluid which runs in the cleft between the buttocks of the wearer.

As mentioned above, a person has essentially the same dimension M throughout his or her life. Articles according to illustrative embodiments described above therefore function in principle for both children and adults if the article as a whole is adapted in terms of size.

An article according to the invention in the form of a nappy for children or adults has a superior fit compared with conventional nappies. The presence of the stiffening element means that, when the nappy is put on, it is guided into the correct position on the wearer and that it remains in this position during use of the article.

Figure 14 shows an embodiment in which the stiffening element 6 is rectangular. As in previous illustrative embodiments, a longitudinal hole 620 is arranged in the rear portion of the article. This hole has the same function as described above and, during use of the article, gives rise to a fold, the height of which increases in the backward direction. During use of the article, the fold stabilizes it in the lateral direction, as described above. The same reference numbers have been used in Figure 14 as in previously described illustrative embodiments.

In the embodiment shown in Figure 15, the article has been provided with three elongate holes 620, 620' and 620" in the rear portion, which holes are positioned symmetrically in relation to the width of the article in the embodiment shown here.

The invention is not limited to the illustrative embodiments described above, but a large number of modifications are possible within the scope of the patent claims below.

For example, anatomically shaped stiffening and absorbent elements of the type described above can be arranged in what are known as pant nappies, that is to say where the nappy is integrated into disposable pants.

It has been stated above that the stiffening absorbent element can be made from different materials and from laminates made of one or more material(s). The stiffening absorbent element can also be made from more than one layer and with the extent of the individual layers being different, in which way it is possible for different areas of the stiffening element to have different stiffness.

As mentioned above, the stiffening element can consist of all the material layers and bonding agents included. Different stiffness in different areas of the stiffening element can therefore also be obtained by varying the degree of connection in different areas, for example different quantities of adhesive in different areas and even the absence of adhesive or other bonding agent in different areas between or in individual layers.

The elastic means 16, which is arranged along the hole 620, has been indicated in the illustrative embodiments described above as having been arranged in a pretensioned state. However, in the manufacture of absorbent articles such as sanitary towels, nappies and the like, it is known to arrange a heat-sensitive elastic means in an untensioned state and to tension the elastic by heat treatment. This suitably takes place when the articles are packed.

In the illustrative embodiments described above relating to articles for arrangement inside the crotch portion of briefs, the article is in the majority of the illustrative embodiments provided with permanently arranged wings for attachment of the article to the briefs with the wings folded around the edge portion of the briefs and attached on the outside of the crotch portion. The wings can consist of separate elements which are attached to the rest of the article in connection with the article being put on. The separate wings can be arranged detachably on the rest of the article during manufacture of the article, as a result of which a wearer who does not want to have wings on the article can remove these in connection with putting the article on.

The illustrative embodiments described above which do not have wings can be provided with separate wings either during manufacture or when the article is put on.

## Claims

1. Absorbent article, such as a sanitary towel, a panty liner, an incontinence pad, a nappy or the like, which article has a longitudinal direction and a transverse direction, a front portion (1), a rear portion (2), a crotch portion (3) located between the rear portion and the front portion, an absorbent element and a liquidtight layer (5), and also a stiffening element (6) which is intended to contribute to the three-dimensional shape of the article during its use, **characterized in that** the stiffening element (6) is in a plane state before use of the article, **in that** the stiffening element extends in the longitudinal direction of the article over at least part of the rear portion (2) of the article from the crotch portion (3), **in that** the stiffening element (6) has in the rear portion (2) at least one elongate second through-hole (620) which extends in the longitudinal direction of the article and along the centre line of the article, as a result of which the article is during use provided, by virtue of lateral forces arising in the rear portion of the article, with a fold along the longitudinal direction of the article along said second hole (620), which fold (17) extends into the cleft between the buttocks of the wearer during use of the article and in this way stabilizes the article in position on the wearer.

2. Article according to Claim 1, **characterized in that** the second hole (620) is pointed at its end next to the crotch portion (3), and **in that** the width of the second hole increases continuously from said end in the backward direction, as a result of which the height of the fold (17) increases continuously in the same direction during use of the article.

3. Article according to Claim 2, **characterized in that** said second hole (620) is located symmetrically and forms an angle (β) of between 10 and 120°, preferably between 15 and 40°, at said point.

4. Article according to any one of Claims 1-3, **characterized in that** the stiffening element also extends over the crotch portion (3) and at least part of the way in over the front portion (1), **in that** the stiffening element (6) has a width (M) at the transition (12) between the crotch portion (3) and the front portion (1) which is adapted to the distance between the muscle tendons of the wearer on both sides of the crotch of the wearer in the groin of the latter and which is of the order of 15-45 mm, **in that**, in the front portion (1) of the article, the side edges of the stiffening element (6) diverge in the direction from the crotch portion (3) at least part of the way in over the front portion (1), and **in that** the side edges of the stiffening element (6) form, in the direction from the crotch area, an acute angle (α) with a line in the longitudinal direction of the article.

5. Article according to any one of the preceding claims, **characterized in that** the stiffening element (6) is absorbent and at the same time constitutes the absorbent element, and **in that** it swells during absorption while on the whole retaining its geometry in the transverse direction of the article.

6. Article according to Claim 4 or 5, **characterized in that** said width (M) of the stiffening element (6) at the transition (12) between the crotch portion and the front portion is of the order of 20-35 mm.

7. Article according to Claim 4 or 5, **characterized in that** said width (M) of the stiffening element (6) at the transition (12) between the crotch portion (3) and the front portion (1) is of the order of 25-30 mm.

8. Article according to any one of the preceding claims, **characterized in that** the stiffening element (6) has a stiffness in the dry state of in the order of 1-15 N measured according to ASTM D 4032-82.

9. Article according to any one of the preceding claims, **characterized in that** the stiffening element (6) consists of a dry-formed fibre mat with a density between 0.15 and 0.75 g/cm³ and a weight per unit area of in the order of 100-400 g/m².

10. Article according to Claim 9, **characterized in that** the dry-formed fibre mat is, after compression, mechanically softened to the desired stiffness.

11. Article according to Claim 9 or 10, **characterized in that** the dry-formed fibre mat is provided with the desired reduced stiffness and the desired extensibility by virtue of the degree of compression selected and the compression pattern selected.

12. Article according to any one of Claims 4-11, **characterized in that** the side edges of the stiffening element (6), which diverge at least part of the way from the crotch portion (3) in over the front portion (1) of the article, are arranged so as to form an angle (α) between a line in the longitudinal direction of the article and each of said side edges of in the order of 35-55°, preferably in the order of 45°.

13. Article according to any one of Claims 4-11, **characterized in that** the crotch portion (3) has a length (G) of in the order of 70-120 mm, and **in that** the side edges (18, 19) of the stiffening element diverge in the direction from the crotch portion (3) at least part of the way from the crotch portion in over the rear portion of the article.

14. Article according to any one of the preceding claims, **characterized in that** the stiffening element (6) also constitutes the absorbent element, **in that** it has a stiffness of at least 1.0 N, and **in that** the stiffening element is designed with essentially the same stiffness over the entire extent of the stiffening element.

15. Article according to any one of the preceding claims, **characterized in that** a hump-forming element (24) made of a resilient material is arranged under the absorbent element over at least part of the crotch portion (3), which hump-forming element is arranged so as to bring about a raised portion (240) on the side which is intended to be fitted against the wearer, the raised portion being arranged so as to come to lie directly in front of the genitals of the wearer after fitting of the article on the wearer.

16. Article according to Claim 15, **characterized in that** the raised portion (240) is elongate in the longitudinal direction of the article and has a length of between 20 mm and 120 mm.

17. Article according to Claim 15 or 16, **characterized in that** the raised portion (240) is narrower than the rest of the article in the crotch area, and **in that** the raised portion is at least twice as thick as the surrounding areas.

18. Article according to any one of the preceding claims, **characterized in that** an elastic means (16) is arranged in the longitudinal direction of the article and centrally along the rear portion of the article and along at least part thereof from the crotch portion, which elastic means is intended, along its length, to draw adjacent material portions together and curve the article upwards for better contact with the body of the wearer.

19. Article according to any one of the preceding claims, **characterized in that** the stiffening element (6) serves as an absorption means and has very great liquid-spreading capacity for spreading bodily fluid received in the relatively narrow crotch area (3) bounded by the anatomy of the wearer directly in front of the genitals of the wearer over the absorbent portions of the whole article, and **in that** the stiffening element is designed with great swelling capacity in the depth direction and attendant great absorption capacity.

20. Article according to Claim 18, **characterized in that** the stiffening element (6) also serves as an absorption element and is essentially homogeneous over its entire extent with regard to thickness, stiffness, spreading capacity and absorption capacity, as a result of which the stiffening element (6) and thus also the absorption element curve evenly during use without forming local irregularities which may give rise to undesirable spreading of liquid.

21. Article according to any one of the preceding claims, **characterized in that** the length of said transition (12) between the crotch portion (3) and the front portion (1), at which the width of the stiffening element is adapted to the distance between the muscle tendons of the wearer on both sides of the crotch of the wearer in the groin of the latter, is of the order of 5-15 mm.

22. Article according to any one of Claims 4-21, **characterized in that** the stiffening element (6) also constitutes the absorbent element, and **in that** the width of the stiffening element after said transition (12) increases continuously in the crotch portion (3) in the backward direction towards the rear portion (2) for the purpose of optimally utilizing available width space in this area with regard to maximum absorption.

23. Article according to any one of Claims 4-22, **characterized in that** the article is arranged so as, by virtue of the stiffness selected for the stiffening element (6) and by virtue of the selection of said geometry and dimensions in and around the transition (12) between the crotch portion (3) and the front portion (1), when the article is positioned in connection with it being put on with the transition (12) between the front portion and the crotch portion between said muscle tendons, to be fixed in between these and in this way be transformed from plane form to three-dimensional form with the front portion curved upwards in relation to the crotch portion and forming a bowl-like shape at least in an area next to the crotch portion.

## Patentansprüche

1. Absorptionsartikel, wie zum Beispiel eine Binde, eine Slipeinlage, ein Inkontinenzpad, eine Windel oder dergleichen, welcher Artikel eine Längsrichtung und eine Querrichtung, einen Frontabschnitt (1), einen Rückabschnitt (2), einen Schrittabschnitt (3), der zwischen dem Rückabschnitt und dem Frontabschnitt angeordnet ist, ein Absorptionselement und eine flüssigkeitsdichte Schicht (5) und auch ein Versteifungselement (6) aufweist, das dazu gedacht ist, zu der dreidimensionalen Form des Artikels während seiner Verwendung beizutragen, **dadurch gekennzeichnet, dass** das Versteifungselement (6) vor der Verwendung des Artikels in einem ebenen Zustand ist, dass sich das Versteifungselement in der Längsrichtung des Artikels von dem Schrittabschnitt (3) über zumindest einen Teil des Rückabschnitts (2) des Artikels erstreckt, dass das Versteifungselement (6) in dem Rückabschnitt (2) zumindest eine längliche zweite Durchgangsöffnung (620), die sich in der Längsrichtung des Artikels und entlang der Mittellinie des Artikels erstreckt, aufweist, woraus folgt, dass der Artikel während der Verwendung mit Hilfe lateraler Kräfte, die in dem Rückabschnitt des Artikels entstehen, mit einer Falz entlang der Längsrichtung des Artikels entlang der zweiten Öffnung (620) versehen ist, welche Falz (17) sich in den Spalt zwischen den Gesäßhälften des Benutzers während der Verwendung des Artikels erstreckt und auf diese Weise den Artikel an dem Benutzer in Position hält.

2. Artikel nach Anspruch 1, **dadurch gekennzeichnet, dass** die zweite Öffnung (620) an ihrem Ende neben dem Schrittabschnitt (3) spitz zuläuft und dass die Breite der zweiten Öffnung kontinuierlich von diesem Ende in rückwärtiger Richtung ansteigt, weshalb die Höhe der Falz (17) während der Verwendung des Artikels kontinuierlich in derselben Richtung ansteigt.

3. Artikel nach Anspruch 2, **dadurch gekennzeichnet, dass** die zweite Öffnung (620) symmetrisch angeordnet ist und an diesem Punkt einen Winkel (β) von zwischen 10 und 120°, bevorzugt zwischen 15 und 40° bildet.

4. Artikel nach einem der Ansprüche 1 bis 3, **dadurch gekennzeichnet, dass** das Versteifungselement sich auch über den Schrittabschnitt (3) und zumindest zu einem Teil des Weges in den Frontabschnitt (1) hinein erstreckt, dass das Versteifungselement (6) eine Breite (M) an dem Übergang (12) zwischen dem Schrittabschnitt (3) und dem Frontabschnitt (1) aufweist, die auf den Abstand zwischen den Muskelsträngen des Benutzers auf beiden Seiten des Schritts des Benutzers in der Leiste des letztgenannten angepasst ist und die in der Größenordnung von 15-45 mm ist, dass in dem Frontabschnitt (1) des Artikels die Seitenkanten des Versteifungselements (6) in der Richtung von dem Schrittabschnitt (3) zumindest zu einem Teil des Wegs in den Frontabschnitt (1) hinein divergieren und dass die Seitenkanten des Versteifungselements (6) in der Richtung von dem Schrittbereich einen spitzen Winkel (α) mit einer Linie in der Längsrichtung des Artikels bilden.

5. Artikel nach einem der vorhergehenden Ansprüche, **dadurch gekennzeichnet, dass** das Versteifungselement (6) absorbierend ist und zur selben Zeit das Absorptionselement bildet und dass es während der Absorption anschwillt, während es insgesamt seine Geometrie in der Querrichtung des Artikels beibehält.

6. Artikel nach Anspruch 4 oder 5, **dadurch gekennzeichnet, dass** die Breite (M) des Versteifungselements (6) an dem Übergang (12) zwischen dem Schrittabschnitt und dem Frontabschnitt in der Größenordnung von 20-35 mm ist.

7. Artikel nach Anspruch 4 oder 5, **dadurch gekennzeichnet, dass** die Breite (M) des Versteifungselements (6) an dem Übergang (12) zwischen dem Schrittabschnitt (3) und dem Frontabschnitt (1) in der Größenordnung von 25-30 mm ist.

8. Artikel nach einem der vorhergehenden Ansprüche, **dadurch gekennzeichnet, dass** das Versteifungselement (6) eine Steifigkeit im trockenen Zustand in der Größenordnung von 1-15 N, gemessen gemäß ASTM D 4032-82, aufweist.

9. Artikel nach einem der vorhergehenden Ansprüche, **dadurch gekennzeichnet, dass** das Versteifungselement (6) aus einer trocken geformten Fasermatte mit einer Dichte von zwischen 0,15 und 0,75 g/cm³ und einer Masse pro Einheitsfläche in der Größenordnung von 100-400 g/m² besteht.

10. Artikel nach Anspruch 9, **dadurch gekennzeichnet, dass** die trocken geformte Fasermatte nach einer Kompression mechanisch auf die gewünschte Steifigkeit aufgeweicht ist.

11. Artikel nach Anspruch 9 oder 10, **dadurch gekennzeichnet, dass** die trocken geformte Fasermatte mit Hilfe des gewählten Kompressionsgrads und des gewählten Kompressionsmusters mit der gewünschten reduzierten Steifigkeit und der gewünschten Dehnbarkeit versehen ist.

12. Artikel nach einem der Ansprüche 4-11, **dadurch gekennzeichnet, dass** die Seitenkanten des Versteifungselements (6), die zumindest zu einem Teil des Weges von dem Schrittabschnitt (3) in den Frontabschnitt (1) des Artikels hinein divergieren, so angeordnet sind, dass sie einen Winkel (α) zwischen einer Linie in der Längsrichtung des Artikels und jeder der Seitenkanten in der Größenordnung von 35-55°, bevorzugt in der Größenordnung von 45° bilden.

13. Artikel nach einem der Ansprüche 4-11, **dadurch gekennzeichnet, dass** der Schrittabschnitt (3) eine Länge (G) in der Größenordnung von 70-120 mm aufweist und dass die Seitenkanten (18, 19) des Versteifungselements in der Richtung von dem Schrittabschnitt (3) zumindest zu einem Teil des Weges von dem Schrittabschnitt in den Rückabschnitt des Artikels hinein divergieren.

14. Artikel nach einem der vorhergehenden Ansprüche, **dadurch gekennzeichnet, dass** das Versteifungselement (6) auch das Absorptionselement bildet, dass es eine Steifigkeit von zumindest 1,0 N aufweist und dass das Versteifungselement über die gesamte Ausdehnung des Versteifungselements im Wesentlichen mit derselben Steifigkeit ausgestaltet ist.

15. Artikel nach einem der vorhergehenden Ansprüche, **dadurch gekennzeichnet, dass** ein eine Aufwölbung bildendes Element (24), das aus einem elastischen Material hergestellt ist, über zumindest einen Teil des Schrittabschnitts (3) unter dem Absorptionselement angeordnet ist, welches eine Aufwölbung bildende Element so angeordnet ist, dass es einen erhöhten Abschnitt (240) an der Seite, die dazu gedacht ist, an den Benutzer angefügt zu werden, herbeiführt, wobei der erhöhte Abschnitt so angeordnet ist, dass er nach dem Anfügen des Artikels an den Benutzer direkt vor den Genitalien des Benutzers zu liegen kommt.

16. Artikel nach Anspruch 15, **dadurch gekennzeichnet, dass** der erhöhte Abschnitt (240) in der Längsrichtung des Artikels erstreckt ist und eine Länge von zwischen 20 mm und 120 mm aufweist.

17. Artikel nach Anspruch 15 oder 16, **dadurch gekennzeichnet, dass** der erhöhte Abschnitt (240) schmaler als der Rest des Artikels in dem Schrittbereich ist und dass der erhöhte Abschnitt zumindest doppelt so dick wie die umgebenden Bereiche ist.

18. Artikel nach einem der vorhergehenden Ansprüche, **dadurch**
**gekennzeichnet, dass** ein elastisches Mittel (16) in der Längsrichtung des Artikels und zentral entlang dem Rückabschnitt des Artikels und zumindest entlang einem Teil von diesem von dem Schrittabschnitt aus angeordnet ist, welches elastische Mittel dazu gedacht ist, entlang seiner Länge benachbarte Materialabschnitte zusammenzuziehen und den Artikel für einen besseren Kontakt mit dem Körper des Benutzers nach oben zu krümmen.

19. Artikel nach einem der vorhergehenden Ansprüche, **dadurch gekennzeichnet, dass** das Versteifungselement (6) als ein Absorptionsmittel dient und eine sehr große Flüssigkeitsverteilungskapazität zum Verteilen eines Körperfluids, das in dem relativ schmalen Schrittbereich (3) aufgenommen wird, der durch die Anatomie des Benutzers direkt vor den Genitalien des Benutzers über den Absorptionsabschnitten des gesamten Artikels begrenzt ist, aufweist und dass das Versteifungselement mit einer großen Schwellkapazität in der Tiefenrichtung und einer dazugehörigen großen Absorptionskapazität ausgestaltet ist.

20. Artikel nach Anspruch 18, **dadurch gekennzeichnet, dass** das Versteifungselement (6) auch als ein Absorptionselement dient und im Wesentlichen über seine gesamte Erstreckung in Bezug auf Dicke, Steifigkeit, Verteilungskapazität und Absorptionskapazität homogen ist, weshalb sich das Versteifungselement (6) und damit auch das Absorptionselement während der Verwendung gleichmäßig krümmen, ohne lokale Irregularitäten zu bilden, die eine ungewünschte Verteilung von Flüssigkeit herbeiführen könnten.

21. Artikel nach einem der vorhergehenden Ansprüche, **dadurch gekennzeichnet, dass** die Länge des Übergangs (12) zwischen dem Schrittabschnitt (3) und dem Frontabschnitt (1), an dem die Breite des Versteifungselements auf den Abstand zwischen den Muskelsträngen des Benutzers auf beiden Seiten des Schritts des Benutzers in der Leiste des letztgenannten angepasst ist, in der Größenordnung von 5-15 mm ist.

22. Artikel nach einem der Ansprüche 4-21, **dadurch gekennzeichnet, dass** das Versteifungselement (6) auch das Absorptionselement bildet und dass sich die Breite des Versteifungselements nach dem Übergang (12) kontinuierlich in dem Schrittabschnitt (3) in der rückwärtigen Richtung in Richtung des Rückabschnitts (2) vergrößert, um verfügbaren Raum in der Breite in diesem Bereich in Bezug auf maximale Absorption optimal zu nutzen.

23. Artikel nach einem der Ansprüche 4-22, **dadurch gekennzeichnet, dass** der Artikel so angeordnet ist, dass er mit Hilfe der für das Versteifungselement (6) gewählten Steifigkeit und mit Hilfe der Auswahl der Geometrie und Ausmaße in und um den Übergang (12) zwischen dem Schrittabschnitt (3) und dem Frontabschnitt (1) zwischen diesen befestigt wird und auf diese Weise von einer ebenen Form in eine dreidimensionale Form überführt wird, wobei der Frontabschnitt in Bezug auf den Schrittabschnitt nach oben gekrümmt ist und zumindest in einem Bereich neben dem Schrittabschnitt eine becherähnliche Form bildet, wenn der Artikel mit dem Übergang (12) zwischen dem Frontabschnitt und dem Schrittabschnitt zwischen den Muskelsträngen angesetzt und positioniert wird.

## Revendications

1. Article absorbant tel qu'une serviette hygiénique, un protège-slip, une protection contre l'incontinence, une couche ou un article similaire, lequel article présente une direction longitudinale et une direction transversale, une partie avant (1), une partie arrière (2), une partie d'entrejambe (3) située entre la partie arrière et la partie avant, un élément absorbant et une couche étanche aux liquides (5) ainsi qu'un élément de raidissement (6) qui est destiné à contribuer à l'obtention de la forme tridimensionnelle de l'article pendant l'utilisation de celui-ci, **caractérisé en ce que** l'élément de raidissement (6) est à l'état plan avant l'utilisation de l'article, **en ce que** l'élément de raidissement s'étend dans la direction longitudinale de l'article sur au moins une portion de la partie arrière (2) de l'article depuis la partie d'entrejambe (3), et **en ce que** l'élément de raidissement (6) comporte dans la partie arrière (2) au moins un deuxième trou traversant oblong (620) qui s'étend dans la direction longitudinale de l'article et le long de la ligne médiane de l'article, en conséquence de quoi, lors de l'utilisation, l'article sous l'effet de forces latérales qui se produisent dans la partie arrière de l'article, est muni d'un pli qui s'étend dans la direction longitudinale de l'article, le long dudit deuxième trou (620), lequel pli (17) s'étend dans la raie entre les fesses de l'utilisateur, lors de l'utilisation de l'article et de cette manière, maintient l'article de façon stable en place sur l'utilisateur.

2. Article selon la revendication 1, **caractérisé en ce que** le deuxième trou (620) est effilé à son extrémité proche de la partie d'entrejambe (3) et **en ce que** la largeur du deuxième trou augmente de façon continue depuis ladite extrémité en direction de l'arrière, en conséquence de quoi la hauteur du pli (17) augmente de façon continue dans la même direction, lors de l'utilisation de l'article.

3. Article selon la revendication 2, **caractérisé en ce que** ledit deuxième trou (620) est situé symétriquement et forme audit point un angle (β) compris entre 10° et 120°, de préférence entre 15° et 40°.

4. Article selon l'une quelconque des revendications 1 à 3, **caractérisé en ce que** l'élément de raidissement s'étend également sur la partie d'entrejambe (3) et au moins sur une portion de la partie avant (1), **en ce que** l'élément de raidissement (6) a une largeur (M) à l'emplacement de la transition (12) entre la partie d'entrejambe (3) et la partie avant (1), laquelle largeur est adaptée à la distance entre les tendons des muscles de l'utilisateur des deux côtés de l'entrejambe de l'utilisateur dans l'aine de ce dernier et qui est de l'ordre de 15 à 45 mm, **en ce que** dans la partie avant (1) de l'article, les bords latéraux de l'élément de raidissement (6) divergent dans la direction partant de la partie d'entrejambe (3) sur au moins une portion de la partie avant (1), et **en ce que** les bords latéraux de l'élément de raidissement (6) forment, dans la direction partant de la zone d'entrejambe, un angle aigu (α) avec une ligne s'étendant dans la direction longitudinale de l'article.

5. Article selon l'une quelconque des revendications qui précèdent, **caractérisé en ce que** l'élément de raidissement (6) est absorbant et constitue en même temps l'élément absorbant et **en ce qu'**il gonfle pendant l'absorption tout en conservant globalement sa géométrie dans la direction transversale de l'article.

6. Article selon la revendication 4 ou 5, **caractérisé en ce que** ladite largeur (M) de l'élément de raidissement (6), à l'emplacement de la transition (12), entre la partie d'entrejambe et la partie avant est de l'ordre de 20 mm à 35 mm.

7. Article selon la revendication 4 ou 5, **caractérisé en ce que** ladite largeur (M) de l'élément de raidissement (6), à l'emplacement de la transition (12), entre la partie d'entrejambe (3) et la partie avant (1) est de l'ordre de 25 à 30 mm.

8. Article selon l'une quelconque des revendications qui précèdent, **caractérisé en ce que** l'élément de raidissement (6) a une rigidité à l'état sec qui est de l'ordre de 1 à 15 N, mesurée selon la norme ASTM D 4032-82.

9. Article selon l'une quelconque des revendications qui précèdent, **caractérisé en ce que** l'élément de raidissement (6) consiste en un mat de fibres réalisé à sec dont la masse volumique est comprise entre 0,15 et 0,75 g/cm³ et dont la masse surfacique est de l'ordre de 100 à 400 g/m².

10. Article selon la revendication 9, **caractérisé en ce que** le mat de fibres réalisé à sec est, après compression, assoupli mécaniquement jusqu'à atteindre la rigidité désirée.

11. Article selon la revendication 9 ou 10, **caractérisé en ce que** le mat de fibres réalisé à sec est doté de la rigidité réduite désirée et de l'extensibilité désirée du fait du degré de compression choisi et du motif de compression choisi.

12. Article selon l'une quelconque des revendications 4 à 11, **caractérisé en ce que** les bords latéraux de l'élément de raidissement (6) qui divergent sur au moins une portion de la distance allant de la partie d'entrejambe (3) à la partie avant (1) de l'article, sont agencés de manière à former un angle (α) entre une ligne s'étendant dans la direction longitudinale de l'article et chacun desdits bords latéraux, ledit angle (α) étant de l'ordre de 35° à 55°, de préférence de l'ordre de 45°.

13. Article selon l'une quelconque des revendications 4 à 11, **caractérisé en ce que** la partie d'entrejambe (3) a une longueur (G) qui est de l'ordre de 70 à 120 mm et **en ce que** les bords latéraux (18, 19) de l'élément de raidissement divergent dans la direction partant de la partie d'entrejambe (3) sur au moins une portion de la distance entre la partie d'entrejambe et la partie arrière de l'article.

14. Article selon l'une quelconque des revendications qui précèdent, **caractérisé en ce que** l'élément de raidissement (6) constitue également l'élément absorbant, **en ce qu'**il a une rigidité d'au moins 1,0 N et **en ce que** l'élément de raidissement est conçu de manière à avoir essentiellement la même rigidité sur toute l'étendue de l'élément de raidissement.

15. Article selon l'une quelconque des revendications qui précèdent, **caractérisé en ce qu'**un élément (24) formant une protubérance constitué d'un matériau élastique est agencé sous l'élément absorbant sur au moins une portion de la partie d'entrejambe (3), lequel élément formant une protubérance est agencé de manière à procurer une partie relevée (240) sur le côté qui est destiné à être disposé contre l'utilisateur, la partie relevée étant agencée de manière à venir se placer directement en face des parties génitales de l'utilisateur après la mise en place de l'article sur l'utilisateur.

16. Article selon la revendication 15, **caractérisé en ce que** la partie relevée (240) est oblongue dans la direction longitudinale de l'article et a une longueur comprise entre 20 mm et 120 mm.

17. Article selon la revendication 15 ou 16, **caractérisé en ce que** la partie relevée (240) est plus étroite que le reste de l'article dans la zone de l'entrejambe, et **en ce que** la partie relevée est au moins deux fois plus épaisse que les zones qui l'entourent.

18. Article selon l'une quelconque des revendications qui précèdent, **caractérisé en ce qu'**un moyen élastique (16) est agencé dans la direction longitudinale de l'article et de façon centrale le long de la partie arrière de l'article et au moins sur une portion de celle-ci à partir de la partie d'entrejambe, lequel moyen élastique est destiné, sur sa longueur, à ramener ensemble les parties de matériau adjacentes et à courber l'article vers le haut en vue d'obtenir un meilleur contact avec le corps de l'utilisateur.

19. Article selon l'une quelconque des revendications qui précèdent, **caractérisé en ce que** l'élément de raidissement (6) sert de moyen d'absorption et a une très grande capacité d'étalement des liquides en vue de l'étalement, sur les parties absorbantes de l'ensemble de l'article, du fluide corporel reçu dans la zone de l'entrejambe (3) relativement étroite en raison de l'anatomie de l'utilisateur, directement en face des parties génitales de l'utilisateur et **en ce que** l'élément de raidissement est conçu en lui conférant une grande capacité de gonflement dans la direction de la profondeur et une capacité d'absorption importante qui s'en suit.

20. Article selon la revendication 18, **caractérisé en ce que** l'élément de raidissement (6) sert également d'élément absorbant et est essentiellement homogène sur toute son étendue en ce qui concerne l'épaisseur, la rigidité, la capacité d'étalement et la capacité d'absorption, en conséquence de quoi l'élément de raidissement (6) et de ce fait également l'élément absorbant se courbent uniformément lors de l'utilisation sans former des irrégularités locales qui pourraient susciter un étalement indésirable de liquide.

21. Article selon l'une quelconque des revendications qui précèdent, **caractérisé en ce que** la longueur de ladite transition (12) entre la partie d'entrejambe (3) et la partie avant (1), au niveau de laquelle la largeur de l'élément de raidissement est adaptée à la distance entre les tendons des muscles de l'utilisateur des deux côtés de l'entrejambe de l'utilisateur dans l'aine de ce dernier, est de l'ordre de 5 à 15 mm.

22. Article selon l'une quelconque des revendications 4 à 21, **caractérisé en ce que** l'élément de raidissement (6) constitue également l'élément absorbant et **en ce que** la largeur de l'élément de raidissement augmente de façon continue, après ladite transition (12) dans la partie d'entrejambe (3), en direction de l'arrière, vers la partie arrière (2) dans le but d'utiliser de façon optimale l'espace de largeur disponible dans cette zone en vue d'obtenir une absorption maximum.

23. Article selon l'une quelconque des revendications 4 à 22, **caractérisé en ce que** l'article est agencé de telle manière qu'en raison de la rigidité choisie pour l'élément de raidissement (6) et qu'en raison du choix de ladite géométrie et des dimensions dans et autour de la transition (12) entre la partie d'entrejambe (3) et la partie avant (1), lorsque l'article est positionné en étant porté, la transition (12) se situant entre la partie avant et la partie d'entrejambe, entre lesdits tendons des muscles, il soit fixé entre ceux-ci et de cette façon passe d'une forme plane à une forme tridimensionnelle, la partie avant étant courbée vers le haut par rapport à la partie d'entrejambe et adopte une forme de coupe au moins dans une zone proche de la partie d'entrejambe.
